# EUROPEAN PATENT APPLICATION

(11) **EP 3 988 036 A1**
(43) Date of publication of application: **27.04.2022**
(21) Application number: 20826037.2
(22) Date of filing: 18.06.2020
(51) Int. Cl.: A61B 17/125, A61B 17/28

(54) **CUTTER, FORCEPS, SURGICAL SYSTEM, MEDICAL SYSTEM, ROBOT, MEDICAL ROBOT FOR SURGERY, AND SURGICAL SYSTEM**

(30) Priority: 20.06.2019 US 201962864224 P
(71) Applicant: National University Corporation Shiga University of Medical Science, Otsu-shi Shiga 520-2192 (JP)
(72) Inventor: TANI, Tohru, Otsu-shi, Shiga 520-2192 (JP); NAKA, Shigeyuki, Otsu-shi, Shiga 520-2192 (JP); YAMADA, Atsushi, Otsu-shi, Shiga 520-2192 (JP); NAKAKUBO, Tsuyoshi, Kyoto-shi, Kyoto 604-8262 (JP)
(74) Representative: Gunzelmann, Rainer
(86) International application number: PCT/JP2020/023914
(87) International publication number: WO 2020/256053

(57) **Abstract**

[Object] It is an object of the present invention to provide a cutting device, forceps, a surgical system, a medical system, a robot, a surgical medical robot, and a surgical system capable of contacting (gripping) a target portion by a series of operations and cutting the target portion.

[Solution] A cutting device 1 includes rotates a first contact member 14 and a second contact member 116 assembled so as to be openable and closable, a contact mechanism 17 that rotates the first contact member 14 toward the second contact member 116 to bring the first contact member 14 and the second contact member 116 into contact with the target portion B, and a contact mechanism 17 that brings the first contact member 14 and the second contact member 116 into contact with the target portion B, and a cutting mechanism 10 for cutting the target portion B in a state where the contact mechanism 17 causes the first contact member 14 and the second contact member 116 to come into contact with the target portion B.

## Description

### [Technical Field]

The present invention relates to a cutting device that contacts (abuts) and cuts a target site (portion), in particular, forceps, surgical systems, medical systems, robots, surgical medical robots, and surgical systems for performing contacting (grasping or crashing), incision, peeling, coagulation (hemostatic), and amputation surgery operation.

### [Background of the Invention]

The basic operation of excision surgery is repeated coagulation (hemostatic) and amputation operations following exfoliation. During that time, it is also necessary to perform an operation of grasping the target portion. In surgery, it is desirable that each operation be performed continuously, and ideally if the operation may be continued without changing the device.

However, Rigasure, which is famous for its high-frequency energy, has a blade that grips the living tissue (target portion) with a wide blade like a waterfowl's beak, and coagulates by passing a high frequency, and the coagulated part with a cut margin is cut by a scalpel or like scissors with a two-stage configuration and a two-stage operation.

More specifically, as described in the patent document below, there is proposed a scissors-shaped mechanical forceps of bipolar forceps including a pair of openable and closable shaft members with a surgical handle, each of the shaft members having a jaw member with existing electrodes extending from its distal end, wherein the handle is arranged to cooperate to grip the tissue between the jaw members, the electrodes constitute an electrode assembly adapted to connect to an energy source to enable selective conduction of electrosurgical energy, further including a knife actuating mechanism with a trigger configured to selectively advance the knife blade provided to cut the tissue gripped between the jaw members.

With this forceps, the tissue may be grasped by the jaw member and coagulated (hemostatic) by grasping the handle, but in order to cut the grasped coagulated tissue, a manual operation is required to move a trigger separately provided from the handle operation to advance the knife blade.

### [Prior Art Document]

### [Patent Literature]

[Patent Literature 1] Japanese Unexamined Patent Publication No. 2017-060846

### [Summary of the Invention]

### [Problems to be solved by the Invention]

No instrument has been developed that can contact and cut tissue in a series of operations, and a multifunctional device is required for continuous smooth progress of surgical operation and time saving by exchanging instruments. It is especially important in arthroscopic surgery and robotic surgery that are out of the reach of the surgeon. There is a demand for a device that can cut while ensuring coagulation and hemostatic functions and can also grip tissues.

An object of the present invention is to provide a cutting device capable of cutting (grasping) a target portion by a series of operations and cutting the target portion. Furthermore, it is an object of the present invention to provide a forceps, a surgical system, a medical system, a robot, a surgical medical robot, and a surgical system that can contact and cut a tissue by a series of operations.

### [Means for solving problems]

A cutting device of the present invention for solving the above-mentioned problems includes a first contact member and a second contact member assembled so as to be openable and closable, a contacting mechanism that rotates the first contact member toward the second contact member to bring the first contact member and the second contact member into contact with a target portion (site), and a cutting mechanism for cutting the target portion in a state where the first contact member and the second contact member are in contact with the target portion by the contacting mechanism.

According to this configuration, by a series of operations of the contacting (gripping or crashing) mechanism and the cutting mechanism, the contacted target portion can be contacted, and the contacted target portion can be cut without the need for an independent operating member. A cutting member may be provided in the cutting mechanism, and the cutting member may be rotated in the same direction as the rotational drive of the first gripping member and brought into contact with the second gripping member to cut the target portion.

Further, the first gripping member may be provided with a protrusion restricting portion for restricting the cutting member rotating in the same direction from protruding beyond the first gripping member in the direction opposite to the cutting direction. Further, in the cutting mechanism, the cutting member may be configured to slid forward in parallel with the second gripping member to cut the target portion by a cutting edge of the cutting member.

According to an example of the present invention, the cutting device is a forceps which includes the cutting device in which the target portion is a living tissue, and the first gripping member and the second gripping member are a first jaw member and a second jaw member, respectively, a driving mechanism that grips (or crash) and coagulates the biological tissue by rotating the first jaw member toward the second jaw member, and an operation unit for operating the driving mechanism and the cutting mechanism, wherein the living tissue is gripped and coagulated by the first jaw member and the second jaw member by a series of operations of the operation unit to be cut by the cutting mechanism.

The cutting device and the forceps may be configured to include electrodes for irradiating electromagnetic waves which are disposed to at least one of the gripping point that grips the target portion in the first gripping member and the vicinity of the cutting blade in the cutting member, and the gripping point that grips the target portion in the second gripping member. The electrodes may be branched and connected to a coaxial cable that supplies electromagnetic waves.

The cutting device or the forceps may include a first cutting member and a second cutting member provided with a cutting blade for cutting the target portion, and an electrode for irradiating an electromagnetic wave is provided along each of the cutting blades, wherein the electrode may be branched and connected to a coaxial cable that supplies electromagnetic waves.

The electrodes provided on the first jaw member (or the first cutting member) and the second jaw member (or the second cutting member) may be constructed to be connected in the same polar direction or different polar directions with respect to the coaxial cables branched in parallel from the coaxial cable.

According to an example of the present invention, there are provided a method of rotating the first jaw member toward the second jaw member, and gripping or crashing a biological tissue by the first jaw member and the second jaw member, and a coagulation method by irradiation with an electromagnetic wave, and a cutting method by the above-mentioned attached cutting member.

According to an example of the present invention, there is provided a robot including an input/output unit connected to the cutting device or forceps by wire and/or wirelessly, an input unit that receives an operation signal in real time, an arithmetic unit that executes a program of a predetermined operation based on the operation signal, and an output unit that generates a drive signal that contacts the target portion and/or cuts the target portion by the first contact member and the second contact member of the cutting device based on the output from the arithmetic unit, and further there may be provided a cutting method having a robot arm movement, a tool movement, and a cutting step. A working limb of the robot is equipped with the cutting device having a microwave irradiation function on both blades of the cutting device. The output unit generates an irradiation signal for irradiating the electromagnetic wave from the electrode. Further there is provided a surgical system including a plurality of surgeon consoles for controlling the robot.

### [Advantages of the Invention]

According to the present invention, there may be provided a cutting device, forceps, a surgical system, a medical system, a robot, a surgical medical robot, and a surgical system which are capable of contacting (grasping) a target portion in a series of operations and cutting the target portion.

### [Brief Description of the Drawings]

FIG. 1 is an explanatory view of an open forceps according to a first embodiment of the present invention. FIG. 1 at (a) is a front view of the forceps, FIG. 1 at (b) is a partial plane view of the cutting mechanism in the forceps, and FIG. 1 at (c) is a partial front view of the cutting mechanism of FIG. 1 at (b);
FIG.2 is a rear view of the forceps of FIG. 1;
FIG. 3 is a schematic explanatory view of the forceps of FIG. 1. FIG. 3 at (a) is a side sectional view taken along the line A-A in FIG. 2, FIG. 3 at (b) is a partial vertical sectional view of the forceps, and FIG. 3 at (c) is a sectional view taken along the line B-B in FIG. 3 at (b) with an enlarged view of the electrode part;
FIG. 4 is an explanatory view of a gripped state of the forceps of FIG. 1.

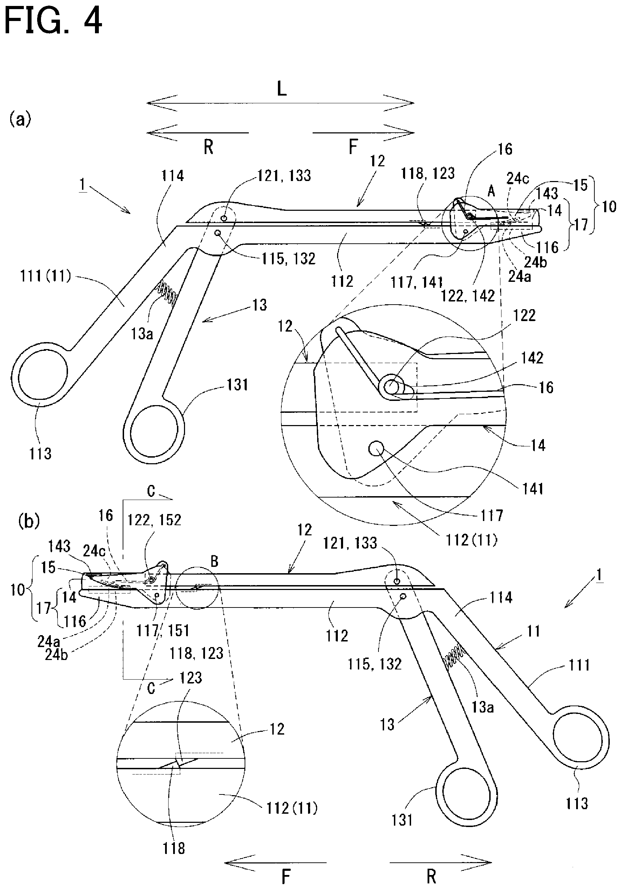

FIG. 4 at (a) is a front view of the gripping state in which the gripping mechanism of the forceps is closed, and FIG. 4 at (b) is a rear view of the forceps in the same state;

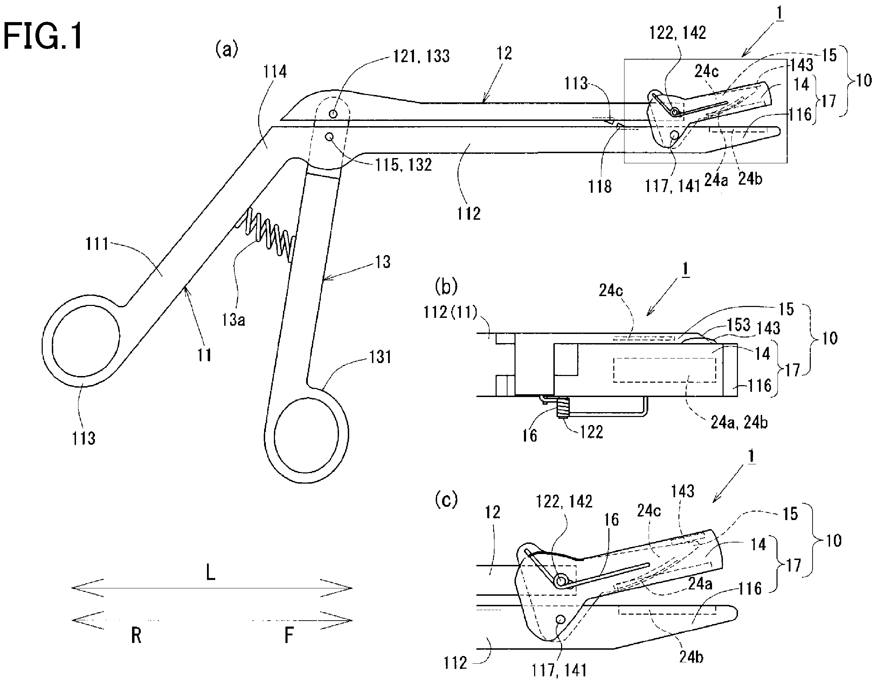

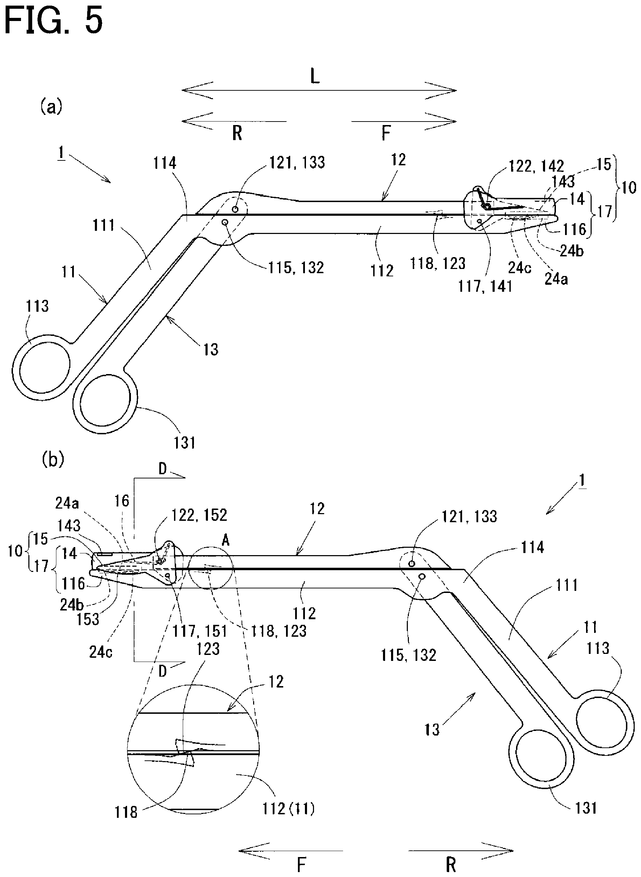

FIG. 5 is an explanatory view of a cut state of the forceps of FIG. 1. FIG. 5 at (a) is a front view of the cutting mechanism of the forceps with the cutter closed, and FIG. 5 at (b) is a rear view of the forceps in the same state;

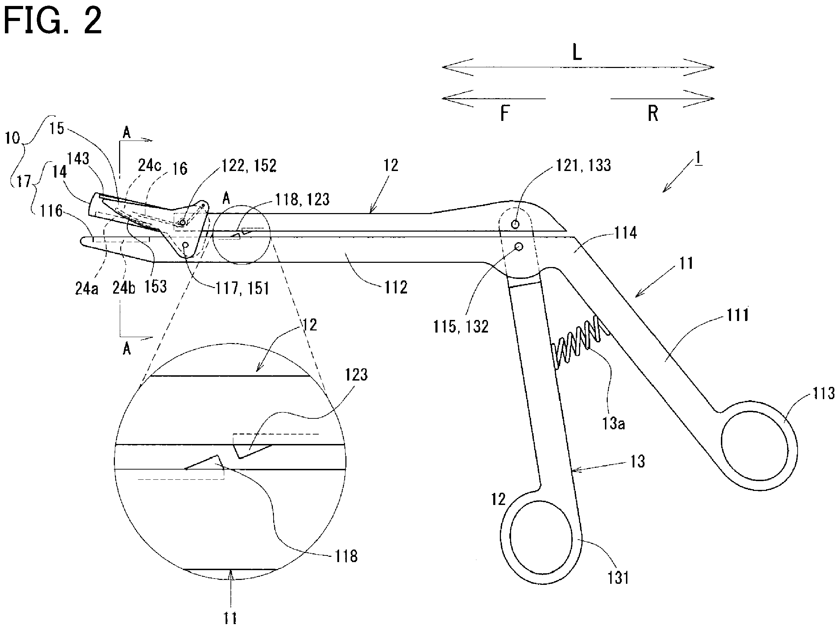

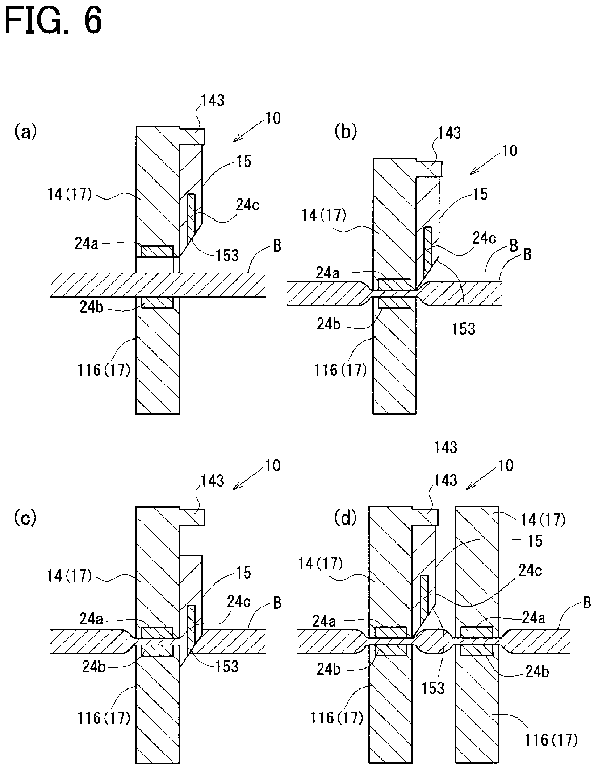

FIG. 6 is an explanatory view of a gripping state and a cutting state of the cutting mechanism in forceps. FIG. 6 at (a) is a side sectional view taken along the line A-A in FIG. 2 before being gripped by the gripping mechanism, FIG. 6 at (b) is a side sectional view taken along the line C-C in FIG. 4 at (b) in a state where the target portion is gripped and crashed by the gripping mechanism, FIG. 6 at (c) is a side sectional view taken along the line D-D in FIG. 5 at (b) in a state where the target portion is cut with a cutter, and FIG. 6 at (d) is a schematic side view on the right in a state of being gripped by a gripping mechanism of a cutting mechanism in another aspect.

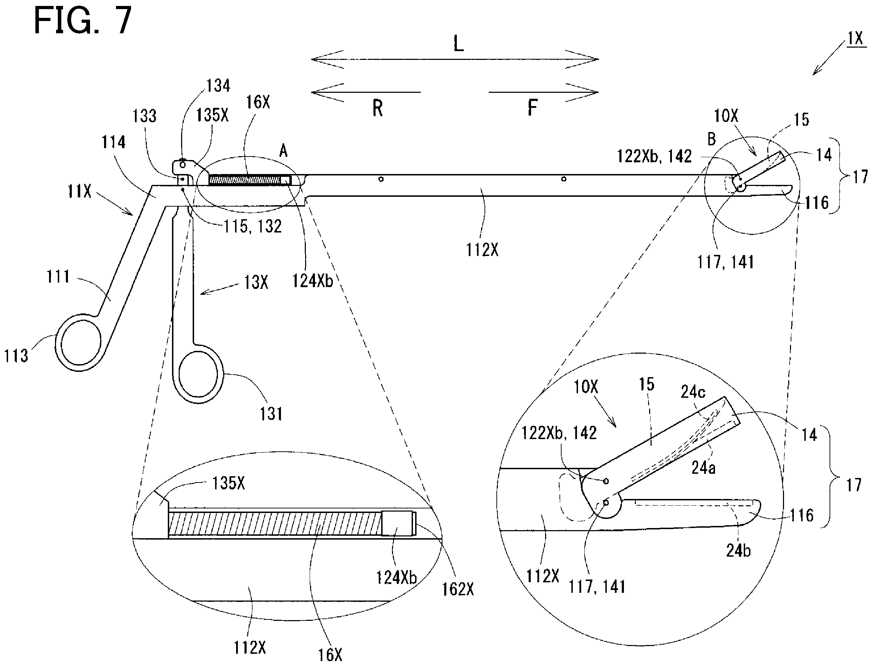

FIG. 7 is a front view of forceps of a second embodiment of the present invention;

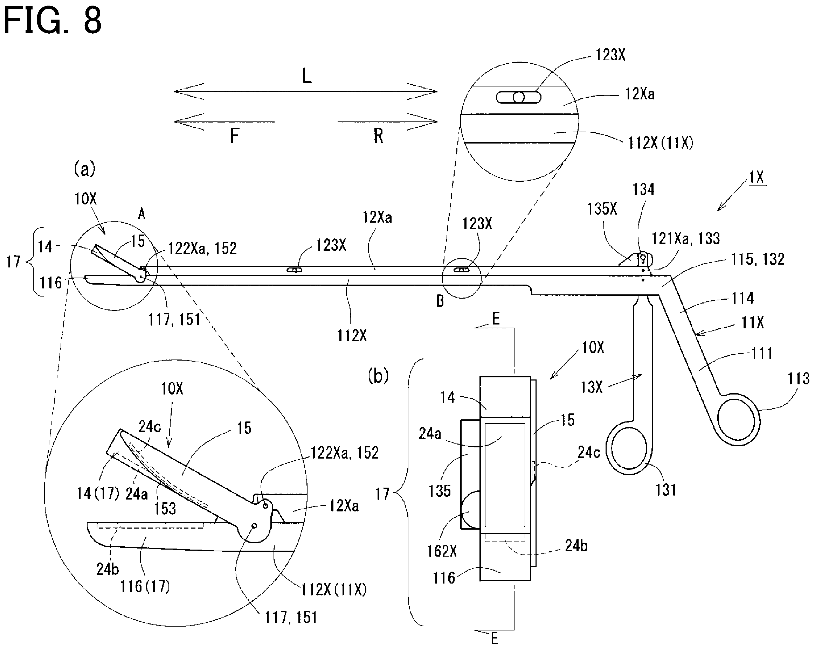

FIG. 8 is an explanatory view of the forceps of FIG. 7. FIG. 8 at (a) is a rear view of the forceps, and FIG. 8 at (b) is a side view of the forceps on the cutting mechanism;

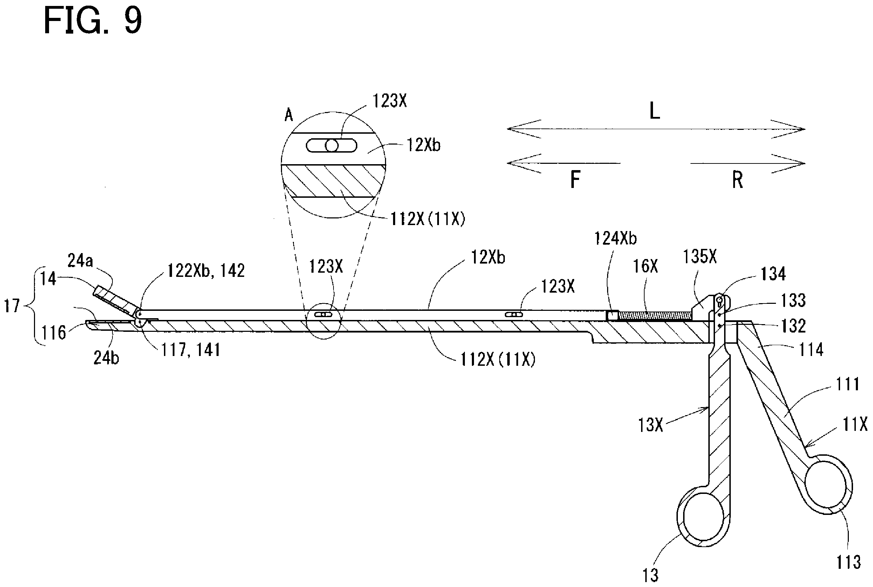

FIG. 9 is a rear view of the forceps of FIG. 8, and a partial cross-sectional view taken along the line E-E in FIG. 8 at (b);

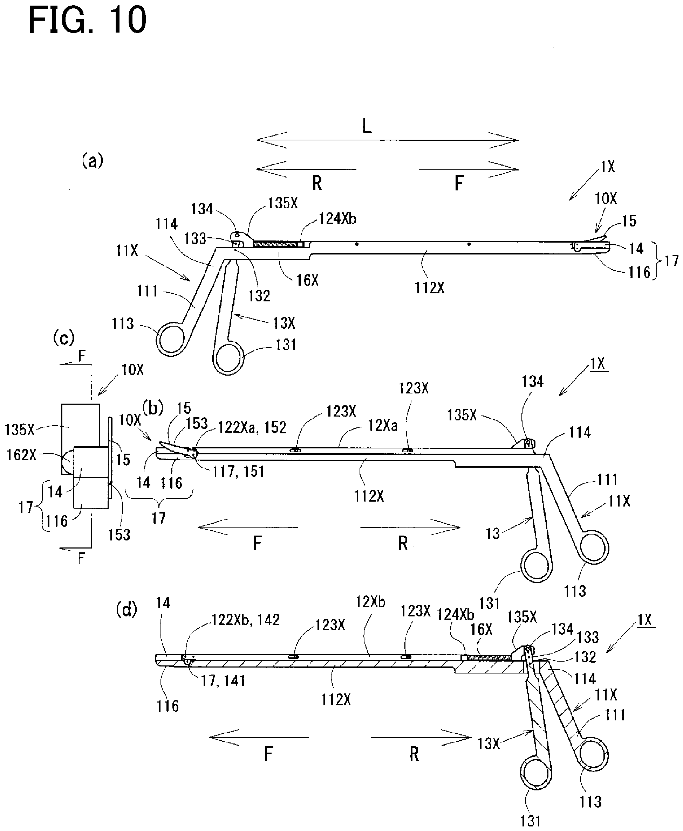

FIG. 10 is an explanatory view of a gripped state of the forceps of FIG. 7. FIG. 10 at (a) is a front view of the forceps in the gripped state, FIG. 10 at (b) is a rear view of the forceps in the same state, FIG. 10 at (c) is a side view of the forceps in the same state on the cutting mechanism side, and FIG. 10 at (d) is a partial cross-sectional view taken along the line F-F in FIG. 10 at (c);

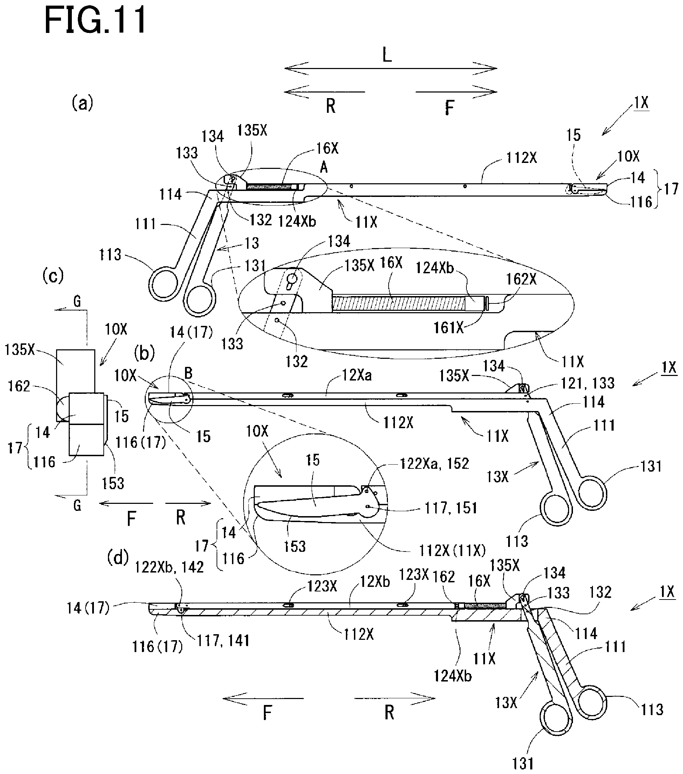

FIG. 11 is an explanatory view of a cut state of the forceps of FIG. 7. FIG. 11 at (a) is a front view of the forceps in the cut state, FIG. 11 at (b) is a rear view of the forceps in the same state, FIG. 11 at (c) is a side view of the forceps in the same state on the cutting mechanism side, and FIG. 11 at (d) is a partial cross-sectional view taken along the line G-G in FIG. 11 at (c);

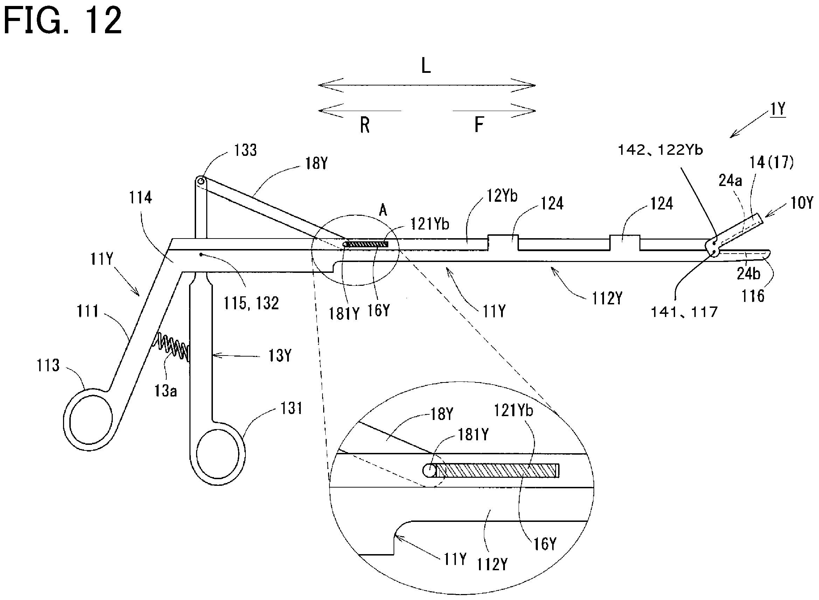

FIG. 12 is a front view of a forceps of a third embodiment of the present invention;

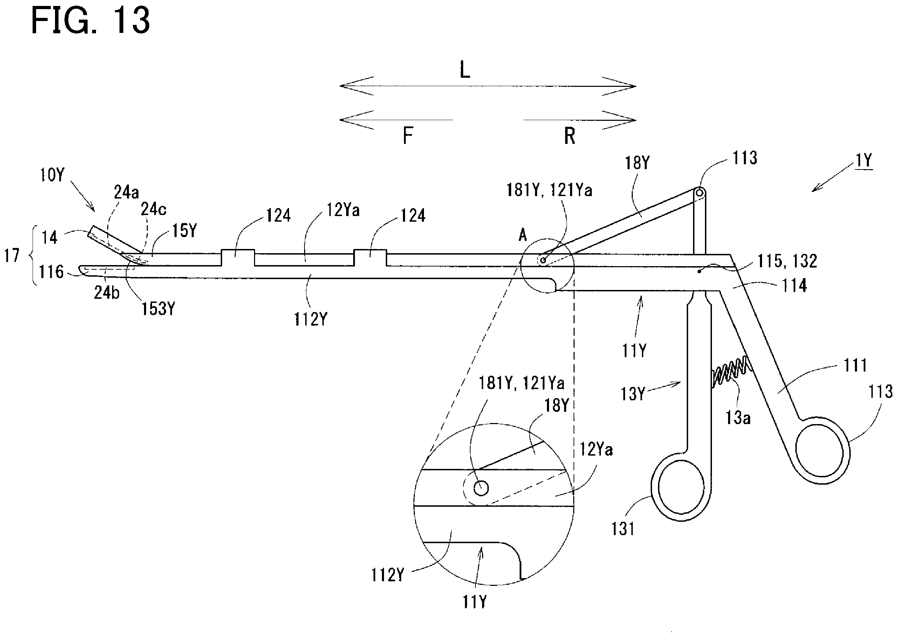

FIG. 13 is a rear view of the forceps of FIG. 12;

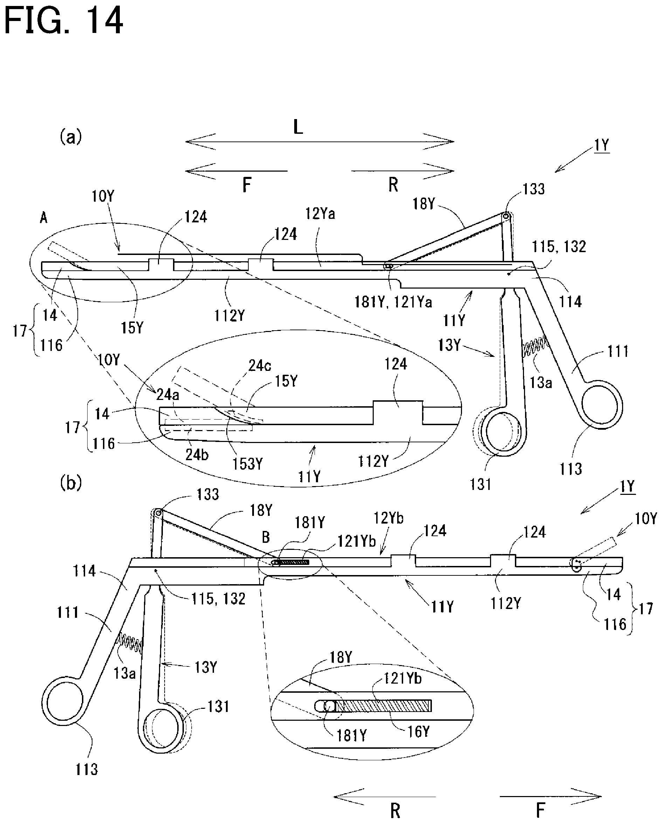

FIG. 14 is an explanatory view of a gripped state of the forceps of FIG. 12. FIG. 14 at (a) is a front view of the forceps in the gripped state, and FIG. 14 at (b) is a rear view of the forceps in the same state;

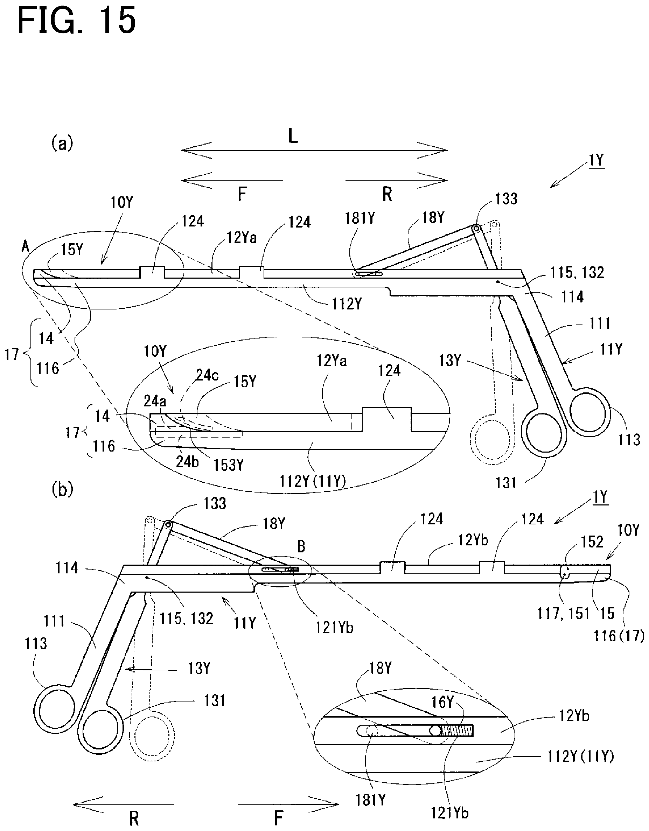

FIG. 15 is an explanatory view of forceps in a cut state. FIG. 15 at (a) is a front view of the forceps in the cut state, and FIG. 15 at (b) is a rear view of the forceps in the same state;

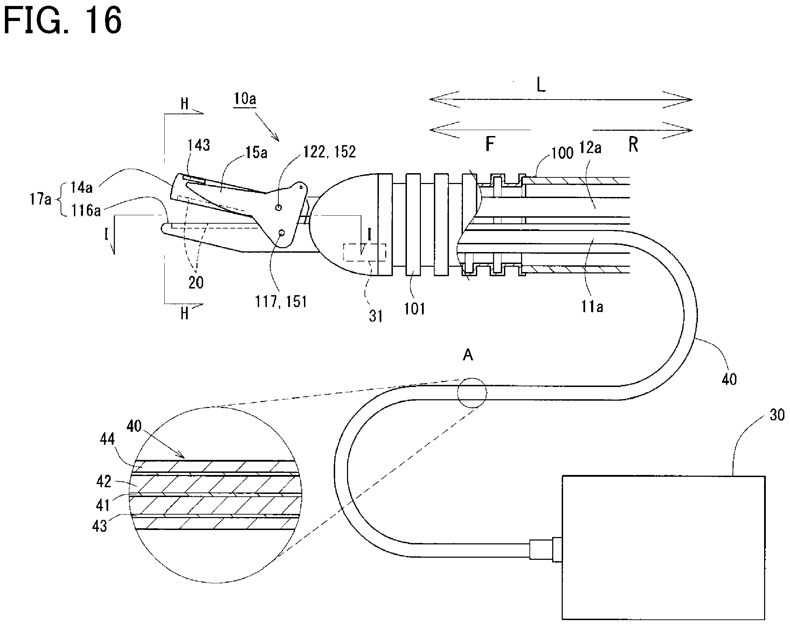

FIG. 16 is a schematic front view of an end effector of a fourth embodiment of the present invention;

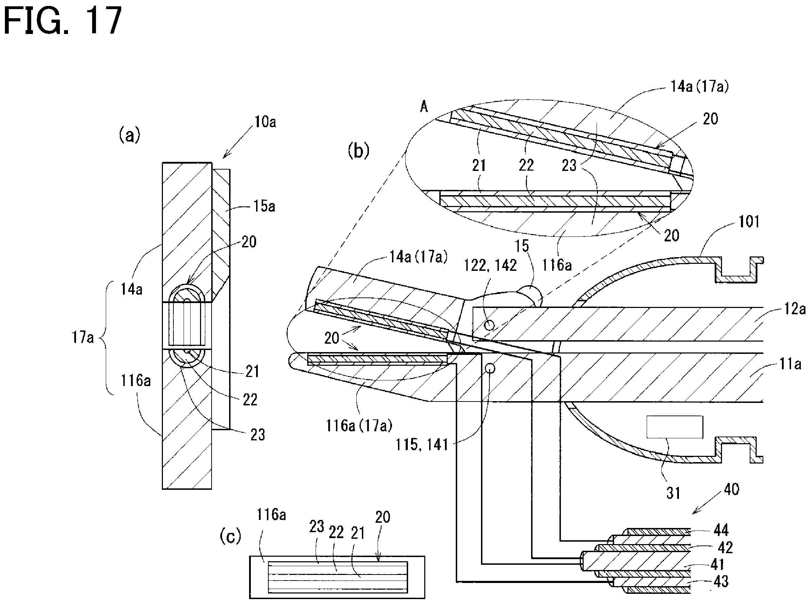

FIG. 17 is a schematic explanatory view of the end effector of FIG. 16. FIG. 17 at (a) is a side sectional view taken along the line H-H in FIG. 16, FIG. 17 at (b) is a partial vertical sectional view of the end effector, and FIG. 17 at (c) is an enlarged plane view of the end effector taken along the line I-I in FIG. 16;

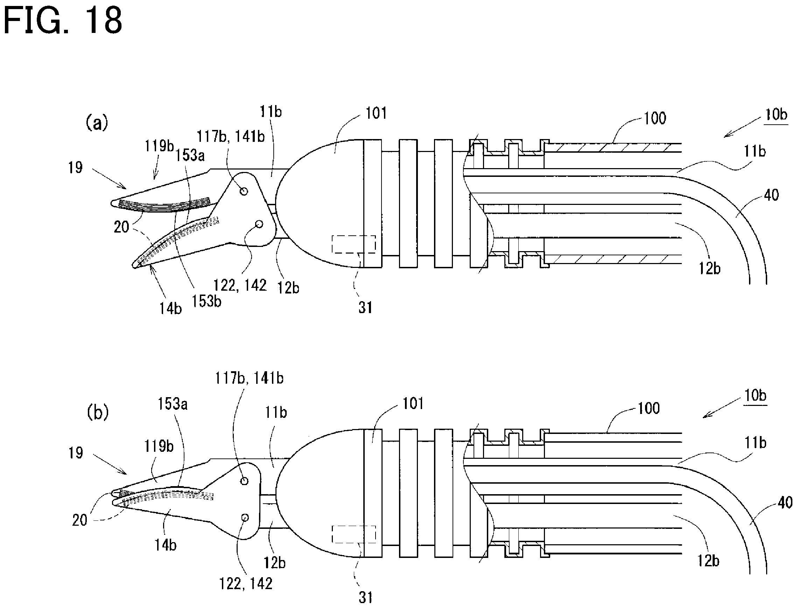

FIG. 18 is a schematic explanatory view of an end effector according to a fifth embodiment of the present invention. FIG. 18 at (a) is a schematic front view of the end effector in the normal state, and FIG. 18 at (b) is a schematic front view of the end effector in the disconnected state;

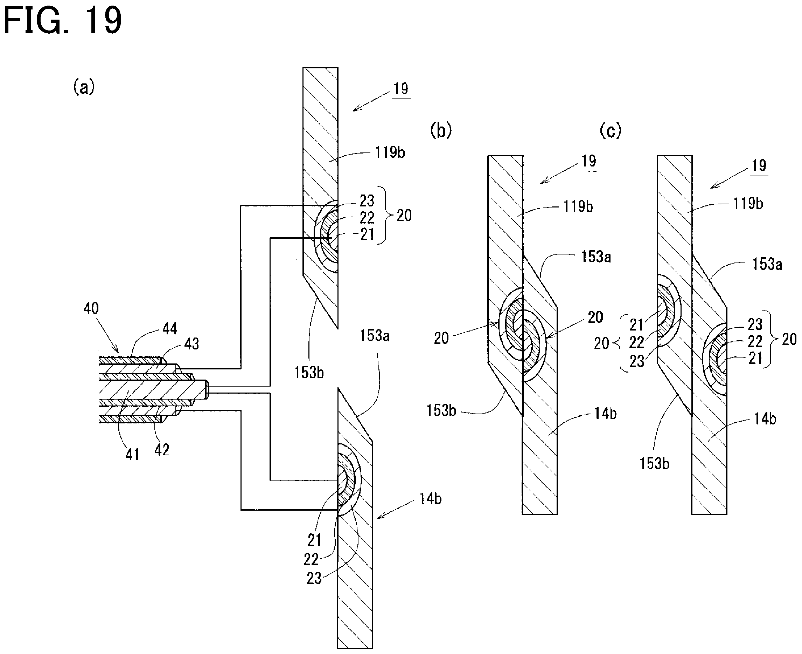

FIG. 19 is a schematic explanatory view of the end effector of FIG. 19 at (a) is a schematic cross-sectional view of a cutting mechanism in an end effector in a normal state, FIG. 19 at (b) is a schematic cross-sectional view of an end effector in a cut state, and FIG. 19 at (c) is schematic cross-sectional view of a cutting mechanism in which the arrangement of coaxial electrodes is different.;

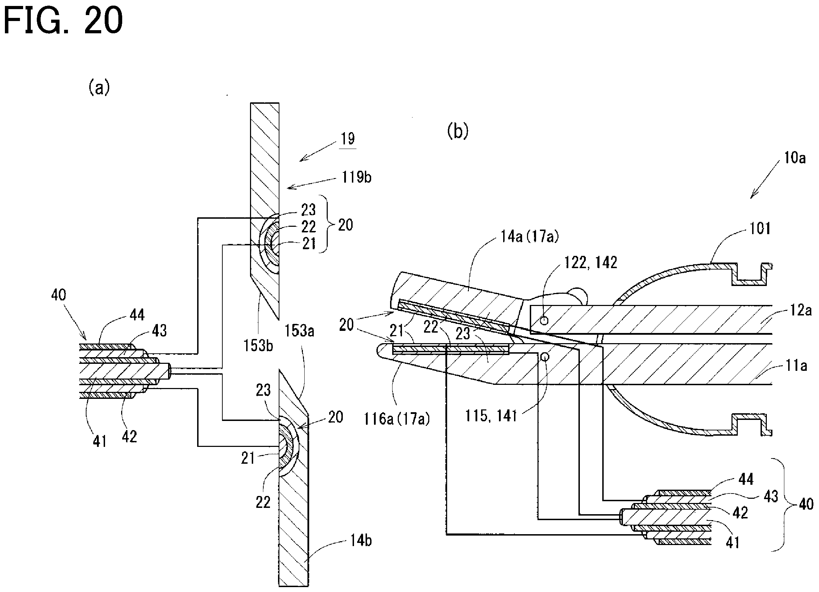

FIG. 20 is a schematic cross-sectional view of an end effector showing an outline of an electrode of a modified connection example of a coaxial electrode and a coaxial cable. FIG. 20 at (a) is a side sectional view of a reverse connection with FIG. 19 at (a), and FIG. 20 at (b) is a partial vertical sectional view of the reverse connection with the connection in FIG. 17 at (b);

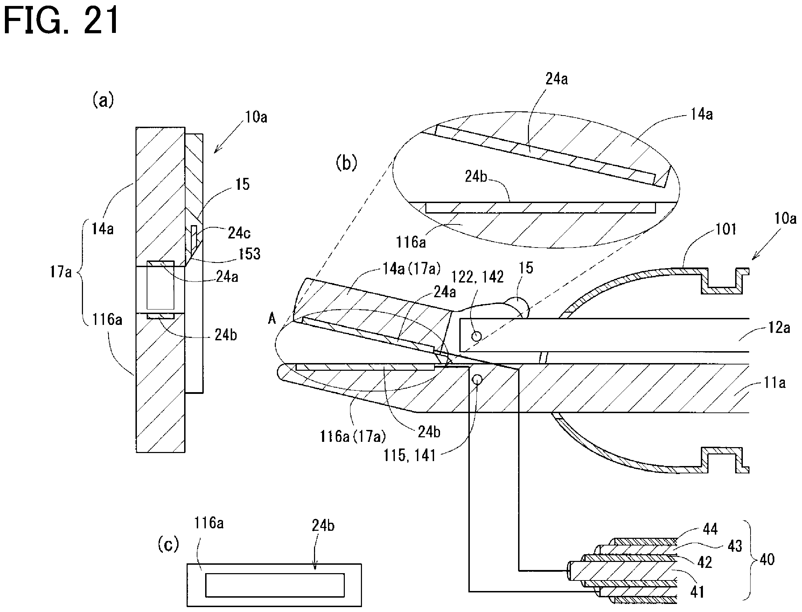

FIG. 21 is a schematic explanatory view of an end effector of a modification of the end effector of FIG. 17. FIG. 21 at (a) is a side sectional view taken along the line H-H in FIG. 16, FIG. 21 at (b) is a partial vertical sectional view of the end effector, and FIG. 21 at (c) is an enlarged plane view taken along the line I-I in FIG. 16;

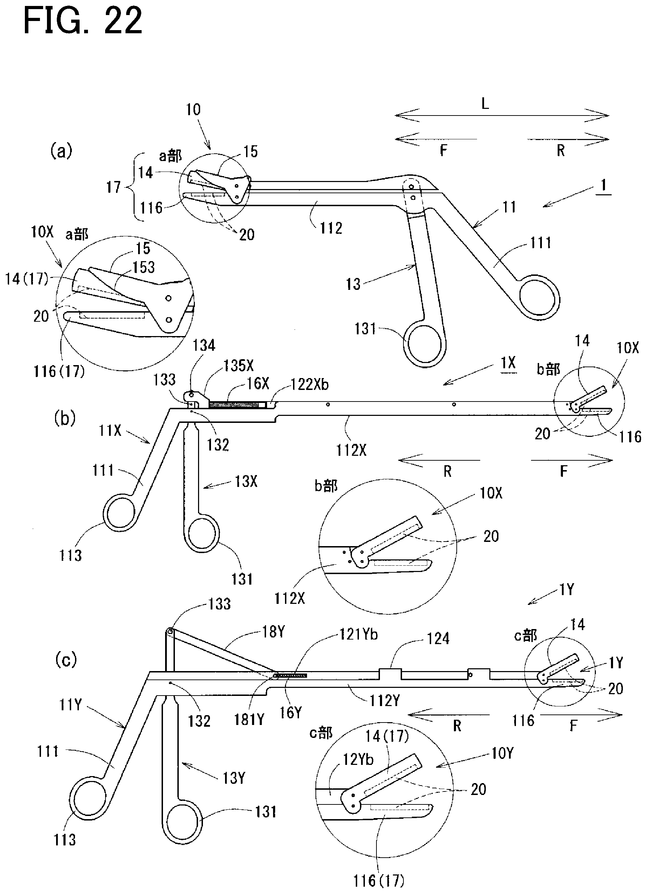

FIG. 22 is a schematic explanatory view of forceps of another embodiment. FIG.22 at (a) is a rear view of the forceps of FIG. 1 having a coaxial electrode, FIG. 22 at (b) is a front view of the forceps of FIG. 7 having a coaxial electrode, and FIG. 22 at (c) is a front view of forceps of FIG. 12 having a coaxial electrode;

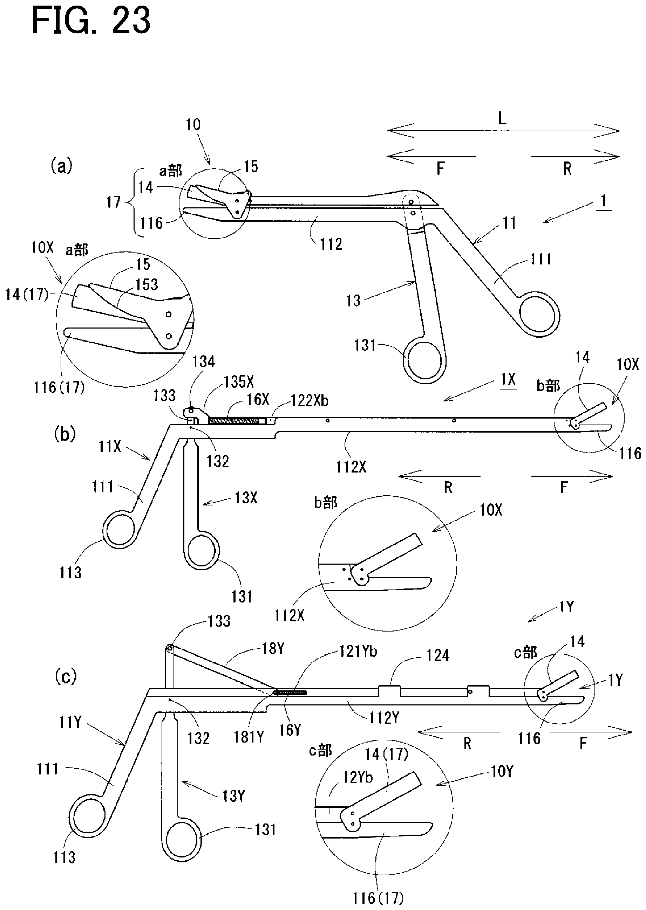

FIG. 23 is a schematic explanatory view of forceps of still another embodiment. 23 at (a) is a rear view of the forceps of FIG. 1 without electrodes, FIG. 23 at (b) is a front view of the forceps of FIG. 7 without electrodes, and FIG. 23 at (c) is a front view of forceps of FIG. 12 without electrodes;

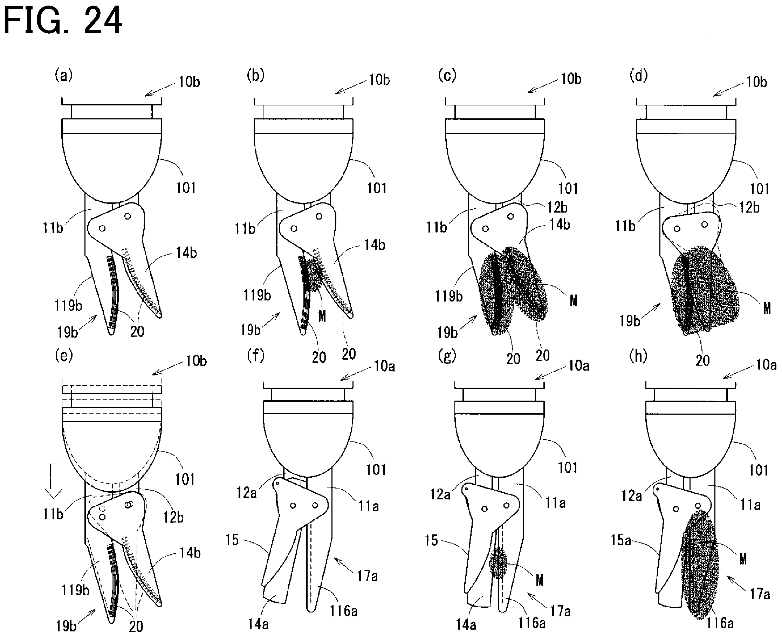

FIG. 24 is an explanatory diagram showing a schematic image of an experimental result by microwave irradiation from an electrode in an end effector;

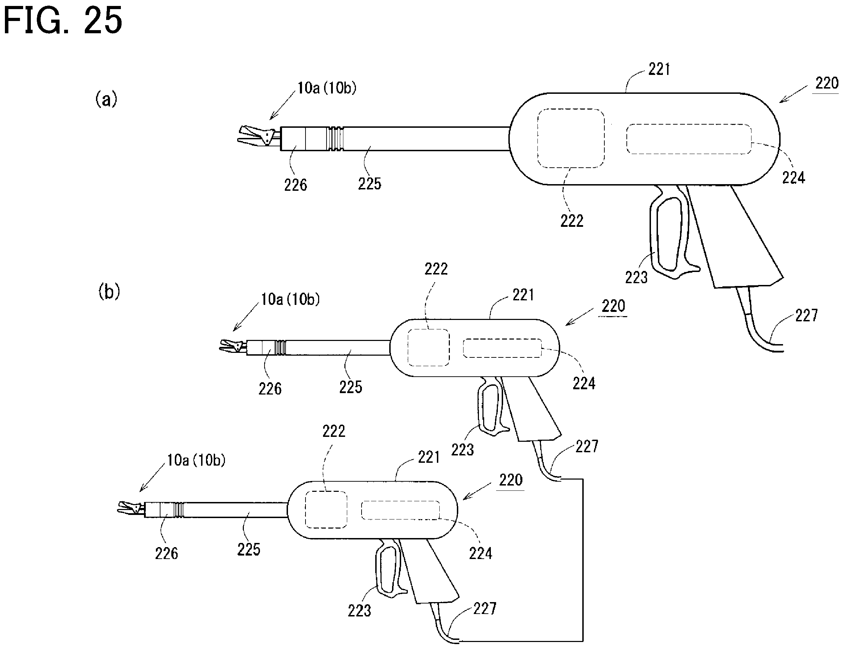

FIG. 25 is a schematic explanatory view of a medical device in another embodiment;

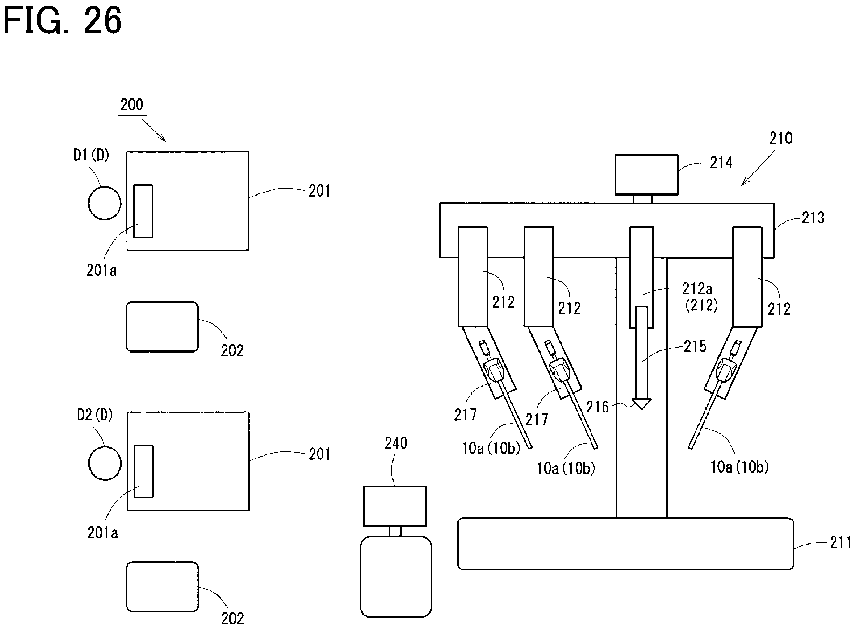

FIG. 26 is a schematic diagram of a remote surgery system in another embodiment;

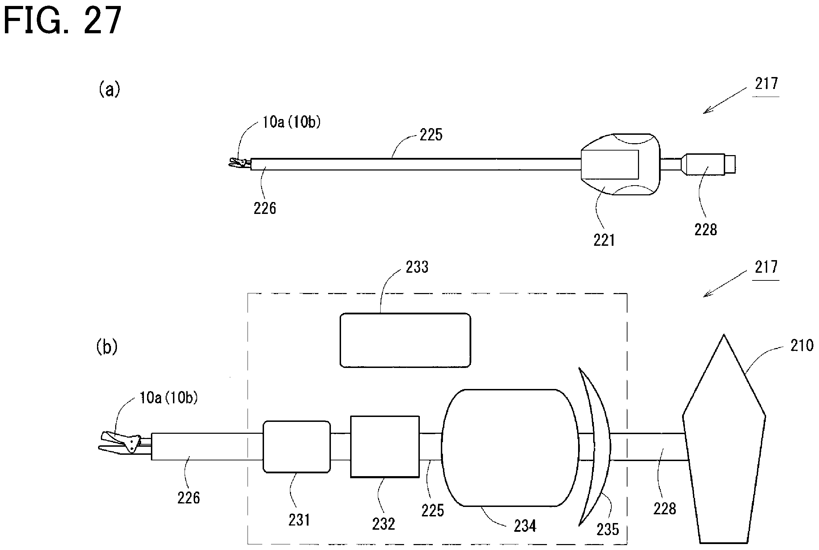

FIG. 27 is a schematic explanatory view of a surgical device in a remote surgery system; and

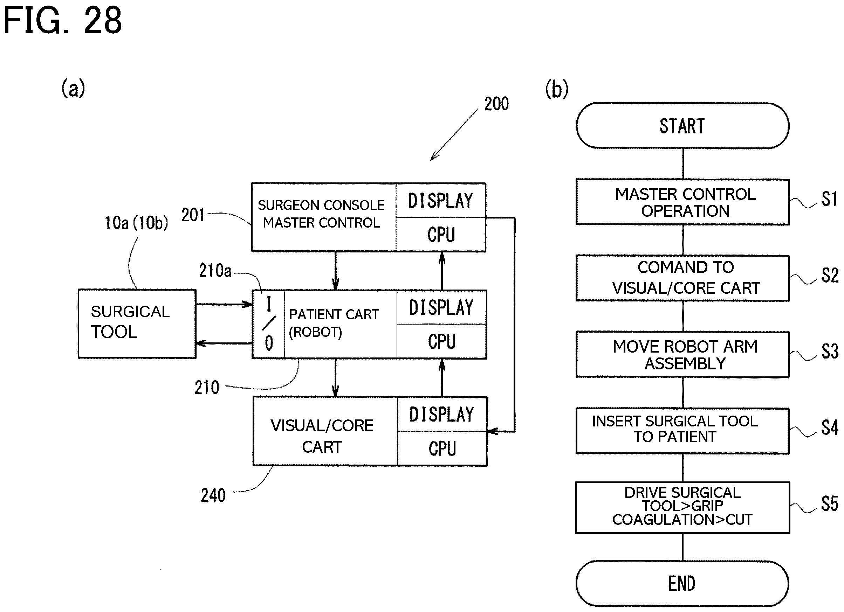

FIG. 28 is an explanatory diagram relating to a remote surgery system.

### [Detailed Description]

Embodiments of the present invention will be described below with reference to the accompanying drawings. In the following description, the same parts and components are designated by the same reference numerals. The present embodiment includes, for example, the following disclosures.

### [Structure 1]

A cutting device, forceps, or medical device (1, 1X, 1Y, 10a, 10b, 220) including a first contact member (14, 14a, 14b) and a second contact member (116, 116a, 119b) assembled so as to be openable and closable, a contact mechanism (17,17a) that rotates the first contact member(14, 14a, 14b) toward the second contact member (116, 116a, 119b) to bring the first contact member (14, 14a, 14b) and the second contact member (116, 116a, 119b) into contact with a target portion (B), and a cutting mechanism (10, 10X, 19) for cutting the target portion in a state where the first contact member (14, 14a, 14b) and the second contact member (116, 116a, 119b) are in contact with the target portion (B) by the contact mechanism (17,17a) . According to this configuration, it is possible to grip an external member such as a plate material, a pipe, a rod, or a tissue and cut the gripped member. In addition, the gripped member can be processed by heating, irradiating with energy such as microwaves, or the like in a gripped/crashed state, and then cut by the cutting member. It can be used in multiple fields as a cutting device.

### [Structure 2]

The cutting device, forceps, or medical device (1, 1X, 1Y, 10a, 220) according to Structure 1, further including a gripping mechanism (17, 17a) in which the first contact member (14,14a) and the second contact member (116, 116a) are a first gripping member(14,14a) and a second gripping member (116,116a), and the contact mechanism (17,17a) rotates the first gripping member (14, 14a) toward the second gripping member (116,116a) to grip the target portion (B) between the first gripping member (14, 14a) and the second gripping member (116,116a), a cutting member (15, 15a, 15Y) attached to the first gripping member (14, 14a), wherein the cutting mechanism (10, 10X, 19) moves (rotate or slide) the cutting member (15, 15a, 15Y) along the first gripping member in a state where the target portion (B) is gripped by the first gripping member (14, 14a) and the second gripping member (116, 116a) by the gripping mechanism (17, 17a), and the target portion (B) is cut by joining the cutting member (15, 15a, 15Y) with the second gripping member (116, 116a), and a gripping/cutting method using the same above device.

### [Structure 3]

The cutting device, forceps, or medical device (1, 1X, 1Y, 10a, 220) according to Structure 1 or 2, including electrodes (20, 24a, 24b, 24c) which are disposed in at least one of a gripping portion that grips the target portion (B) in the first gripping member (14, 14a) and a vicinity of the cutting blade in the cutting member (15, 15a, 15Y), and a gripping portion that grips the target portion (B) in the second gripping member (116, 116a) for irradiating electromagnetic waves. A method of gripping, coagulating, and cutting a living tissue with forceps, a medical device (1, 1X, 1Y, 10a, 10b, 220), and the same device. A method of gripping, crashing, coagulating, or cutting the target portion by the above device, which may be a method of gripping, crashing, and then coagulating the living or biological tissue.

### [Structure 4]

The electrode (20) is a coaxial electrode (20) provided with a center electrode (21) and an outer electrode (23) surrounding the center electrode (21) via an insulator, and irradiates the electromagnetic wave. The coaxial cable (40) connecting the irradiation device (30) and the electrode (20) is branched in parallel to a plurality of coaxial cables (40), and the coaxial cable (40) is connected to the central conductor (41) and the outer conductor (43). The cutting device, forceps, or medical device (1,1X, 1Y, 10a, 10b, 220) and gripping device according to the structure 3 in which each of the electrodes (20) is electrically connected to the same pole or the opposite polarity, and methods of gripping, crashing, coagulating, and cutting.

### [Structure 5]

The forceps (1,1X, 1Y, 10a) provided with the cutting device (1,1X, 1Y) according to any one of structures 2 to 4 in which the first gripping member (14,14a) and the second gripping member (116,116a) are a first jaw member (14,14a) and a second jaw member (116,116a) respectively, including a driving mechanism for gripping and coagulating a target site (B) by rotating the first jaw member (14,14a) toward the second jaw member (116), and one operation member (13) for operating the driving mechanism and the cutting mechanism, wherein by a series of operations of the operation member (13), and a biological tissue (B) as the target portion (B) is gripped by the first jaw member (14,14a) and the second jaw member (116,116a), and then the forceps (1,1X, 1Y, 10a) cut by the cutting mechanism (10, 10X), and a method of gripping, crashing, coagulating, and cutting by using the forceps or its similar equipment.

In the case of this configuration, the gripping mechanism (17, 17a) and the cutting mechanism (10, 10X) are operated together by the operation of the operation member (13) until the grip is performed, and further operation of the operation member (13) is performed. There may be provided a differential mechanism (142, 16X, 16Y) in which the cutting mechanism (10, 10X) is differential from the gripping mechanism (17, 17a) in a gripping state in which the target portion (B) is gripped and crashed. Further, there may be provided a notification unit (118, 123) for notifying the user of the operation of the differential mechanism (142, 16X, 16Y) by a series of operations of the operation member (13).

### [Structure 6]

The cutting device, forceps, or medical device (10b, 220) according to the Structure 1 having the following components; the first contact member and the second contact member are the cutting member (14b) and the second cutting member (119b) provided with cutting blades (153a, 153b) for cutting the target portion (B) at the contact portion to cut the target portion (B); the contact mechanism is the cutting mechanism (19) for cutting the contacted target portion (B) by the cutting member (14b) and the second cutting member (119b); there are provided electrodes (20) for irradiating electromagnetic waves along the cutting blades (153a, 153b) in the first cutting member (14b) and the second cutting member (119b).

### [Structure 7]

The cutting device, forceps, or medical device (10b, 220) according to Structure 6, wherein the electrodes (20) are coaxial electrodes (20) each provided with a center electrode (21) and an outer electrode (23) surrounding the center electrode (21) via an insulator, a coaxial cable (40) connecting the irradiation device (30) and the electrode (20) is branched in parallel to a plurality of coaxial cables (40), and each of the coaxial electrodes (20) is electrically connected to the central conductor and the outer conductor of the coaxial cable (40) with the same pole or the opposite polarity.

### [Structure 8]

A surgical system (200) including the forceps (1,1X, 1Y, 10a, 10b), wherein the electrodes (20) provided on the forceps (1,1X, 1Y, 10a, 10b) are electrodes (20) for microwave irradiation, and each of the forceps (1,1X, 1Y) is provided with a wave irradiation unit, and a period of the microwave applied to each electrode (20) from the coaxial cable (40) is the same.

### [Structure 9]

A robot including an input / output unit (210a) connected to the cutting device (10a, 10b) by wire and/or wirelessly, an input unit (210a) that receives an operation signal in real time, an arithmetic unit (CPU) that executes a predetermined operation program based on the operation signals, and an output unit (210a) that generates a drive signal to contact and/or cut the target portion (B) by the first contact member (14, 14a, 14b) and the second contact member (116, 116a, 119b) of the cutting device (10a, 10b) and/or an irradiation signal that irradiates the electromagnetic wave from the electrodes (20, 24a, 24b, 24c) based on the output from the arithmetic unit.

### [Structure 10]

A robot control method of generating a command by operating the master control unit (202), moving the arm assembly (212) of the robot (210) to a treatment position by the above command, moving the tool (217) attached to the tool (217) to a treatment position and controlling the movement of the cutting device (10a, 10b) attached to the distal end of the tool (217) and the irradiation of electromagnetic waves.

### [Structure 11]

Surgical system (210) with a robot (210) described in Structure 9 which is a patient-side cart (200), including a plurality of surgeon consoles (201) serving as stations for a plurality of operators, a master control unit (202) operated by a plurality of operators.

The following embodiments will be described as being used for a medical device as an example of the cutting device of the present invention, and examples of forceps and the like will be described below, but the present invention provides a cutting device and a tool not limited to the medical device.

Returning to FIG. 1, there is shown an explanatory diagram of forceps 1 in an open state according to a first embodiment of the present invention. FIG. 1 at (a) shows a front view of the forceps 1, FIG. 1 at (b) shows a partial plane view of the cutting mechanism 10 in the forceps 1, and FIG. 1 at (c) shows a partial front view of the cutting mechanism 10 of FIG. 1 at (b).

FIG. 2 shows a rear view of the forceps 1 of FIG. 1

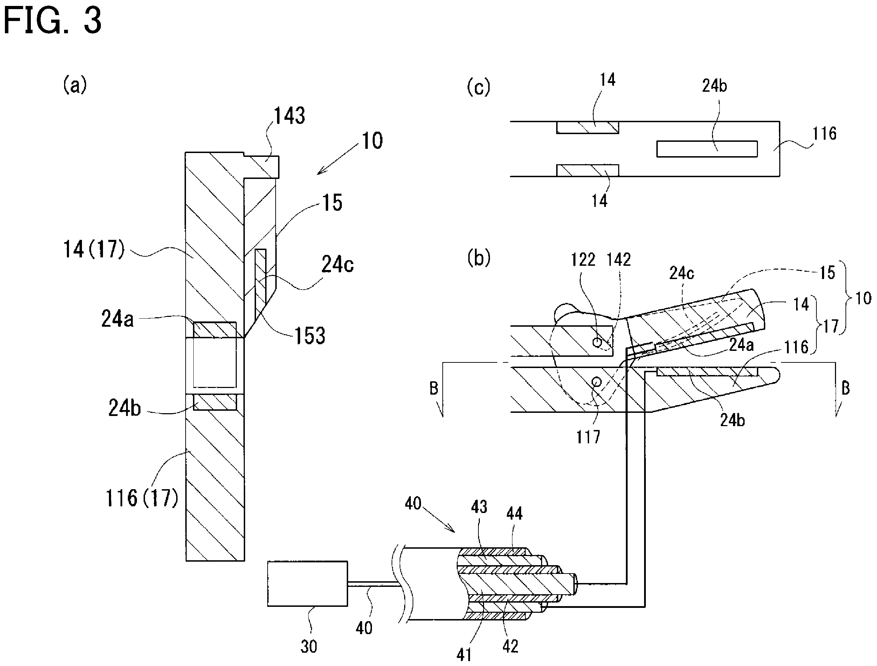

FIG. 3 is a schematic explanatory view of the forceps 1 of FIG. 1. FIG. 3 at (a) is a side sectional view taken along the line A-Ain FIG. 2, FIG. 3 at (b) is a partial vertical sectional view of the forceps, and FIG. 3 at (c) is a sectional view taken along the line B-B in FIG. 3 at (b) with an enlarged view of the electrode part.

FIG. 4 is an explanatory view of a gripped state of the forceps 1 of FIG. 1. FIG. 4 at (a) is a front view of the gripping state in which the gripping mechanism 17 of the forceps is closed, and FIG. 4 at (b) is a rear view of the forceps in the same state.

FIG.5 is an explanatory view of a cut state of the forceps 1 of FIG. 1. FIG. 5 at (a) is a front view of the cutting mechanism 10 of the forceps with the cutter closed, and FIG. 5 at (b) is a rear view of the forceps 1 in the same state.

FIG. 6 is an explanatory view of a gripping state and a cutting state of the cutting mechanism 10 in forceps 1. FIG. 6 at (a) is a side sectional view taken along the line A-Ain FIG. 2 before being gripped by the gripping mechanism 17, and FIG. 6 at (b) is a state in which the target portion is gripped and pressed by the gripping mechanism 17. A side sectional view taken along the line C-C, FIG. 6 at (c) is a side sectional view taken along the line D-D in FIG. 5 at (b) in a state where the target portion is cut with a cutter 15.

In FIG. 1 (FIGS. 1 to 6), the forceps 1 includes a main body frame 11, a slide frame 12, a trigger handle 13, an upper jaw portion 14, the cutter 15, and a spring 16.

The main body frame 11 is roughly composed of two sides that intersect in an obtuse angle, and one of the two sides is a fixed handle frame 111 that functions as an operation member together with the trigger handle 13, and the other is a reference frame 112. The main body frame 11 configured in this way is made of a metal such as stainless steel or a resin.

At the end of the fixed handle frame 111, a ring portion 113 for inserting a user's finger for operating the forceps 1 is provided. If necessary, the ring portion 113 may be provided with a cut portion.

Further, a portion where the fixed handle frame 111 and the reference frame 112 intersect at an obtuse angle is a corner portion 114, and a trigger handle 13 described later is pivotally supported at the base portion (near the above-mentioned corner portion 114) of the reference frame 112 by a support shaft 115.

Further, at the end of the distal end side F of the reference frame 112, a lower jaw portion 116 constituting a gripping mechanism 17 for gripping a target portion such as a blood vessel (B) to be treated is provided in cooperation with the trigger handle 13. The lower jaw portion 116 is configured to have a tapered shape toward the distal end side F in front view. The lower jaw portion 116 is also referred to as "mandibular".

Further, on the distal end side F of the reference frame 112, a tip support shaft 117 that pivotally supports the upper jaw portion 14 and the cutter 15 is provided.

Further, a click convex portion 118 (see FIG. 2) protruding toward the bottom surface of the slide frame 12 is provided on the upper surface of the intermediate portion of the reference frame 112 in the longitudinal direction L.

The click convex portion 118 has a triangular shape in front view protruding toward the bottom surface of the slide frame 12, but has a substantially right angle in front view in which the inclined surface of the base end side R has a steeper inclination angle than the inclined surface of the distal end side F. Further, the click convex portion 118, which has a triangular shape when viewed from the front and protrudes toward the bottom surface of the slide frame 12, is supported so as to bend downward with a predetermined force.

The slide frame 12 is provided so as to be slidable in the longitudinal direction L along the reference frame 112 in the main body frame 11, and is made of the same material as the main body frame 11 and is formed in a prismatic shape.

An upper support shaft 121 that pivotally supports the distal end of the trigger handle 13 is provided on the base end side R (left side in FIG. 1) of the slide frame 12.

Further, on the distal end side (left side in FIG. 1) of the slide frame 12, a distal end top support shaft 122 that pivotally supports the upper jaw portion 14 and the cutter 15 is provided.

Further, a click convex portion 123 (see FIG. 2) protruding toward the upper surface of the reference frame 112 is provided on the bottom surface of the intermediate portion of the slide frame 12 in the longitudinal direction L.

The click convex portion 123 has a triangular shape in front view protruding toward the upper surface of the reference frame 112, but the inclined surface of the distal end side F facing the click convex portion 118 and the longitudinal direction L is from the inclined surface of the proximal end side R. It is formed in the shape of a substantially right-angled triangle in front view, where the inclination angle is steep. Further, the click convex portion 123, which has a triangular shape in front view and protrudes toward the upper surface of the reference frame 112, is supported so as to bend upward with a predetermined force.

The click convex portion 123 is not limited to a triangular shape, but may have another shape such as a polygonal shape or a semicircular shape that provides a function of notifying the user of a click sound or vibration.

The click convex portion 118 and the click convex portion 123 are arranged slightly apart from each other in the longitudinal direction L of the reference frame 112 and the slide frame 12 in the forceps 1 in the initial state (open state).

The trigger handle 13 is an operation member sliding the slide frame 12 along the reference frame 112, and includes a ring portion 131 at the lower end for inserting a user's finger.

Further, in the vicinity of the upper end of the trigger handle 13, a pivot portion 132 that is pivotally supported by the support shaft 115 of the main body frame 11 is provided, and an upper pivot portion 133 is movably supported by the upper support shaft 121 of the slide frame 12 further above the pivot portion 132.

A compression coil spring 13a for automatically returning the slide frame 12 and the trigger handle 13 is provided between the fixed handle frame 111 and the trigger handle 13 (not shown in FIG. 5). The compression coil spring 13a may be omitted depending on the specifications.

The upper jaw portion 14 is provided at the end portion of the distal end side F of the slide frame 12, and constitutes the gripping mechanism 17 together with the lower jaw portion 116 of the main body frame 11. The upper jaw portion 14 is also referred to as "maxilla'..

At the base end portion (left side in FIG. 1) of the upper jaw portion 14, a shaft-bearing portion 141 pivotally supported by the distal end support shaft 117 of the main body frame 11 and a tip-top support shaft of the slide frame 12 above the shaft-mounted portion 141. A rotary bearing portion 142 pivotally supported by the 122 is provided. As shown in FIG. 1 at (c), which is an enlarged view of the portion surrounded by the square in FIG. 1 at (a), the rotary bearing portion 142 has a long hole shape (see FIG. 4) in which the support shaft 122 on the tip is loosely fitted.

A distal end side on the back side of the upper jaw portion 14 is provided with a protrusion control unit 143 to restrict that the cutter 15 rotating along the back surface side surface of the upper jaw portion 14 projects upward from the upper surface of the upper jaw portion 14.

As shown in FIGS. 1 and 2, the cutter 15 is arranged on the back side of the upper jaw portion 14 and is pivotally supported by the distal end support shaft 117 of the main body frame 11 and above the shafted portion 151. A rotation shaft portion 152 that is pivotally supported by a support shaft 122 on the distal end of the slide frame 12 is provided. The cutter 15 configured in this way has a cutting blade 153 formed along the lower end portion. Further, the cutter 15 has a plate shape and is configured to rotate along the side surface of the upper jaw portion 14 with the shafted portion 151 as an axis.

A spring 16 is arranged along the front side of the upper jaw portion 14 and is configured to urge the upper jaw portion 14 and the cutter 15.

More specifically, the spring 16 is a torsion spring that fits outside the support shaft 122 on the distal end, and one arm is fitted to the upper jaw portion 14 and the other arm is fitted to a part of the cutter 15. Therefore, when the upper jaw portion 14 and the cutter 15 are differentially rotated toward the side close to each other with the tip upper support shaft 122 as the center, the spring 16 is attached in the rotation direction in which the upper jaw portion 14 and the cutter 15 are separated from each other.

In the forceps 1 configured in this way, a gripping mechanism 17 that grips the target portion includes the lower jaw portion 116 provided at the end of the distal end side F of the main body frame 11 and the upper jaw portion 14 whose base is pivotally supported by the tip support shaft 117 with respect to the main body frame 11.

Further, the cutter 15 that rotates and cuts the target portion gripped by the gripping mechanism 17 along the side surface of the upper jaw portion 14 constitutes the cutting mechanism 10 together with the gripping mechanism 17.

The forceps 1 is provided with irradiation electrodes 24 (24a, 24b, 24c), and a coaxial cable 40 is connected to the microwave generator 30 (see FIG. 3) that oscillates microwaves and to the irradiation electrodes 24.

Specifically, the upper jaw portion 14 is provided with an irradiation electrode 24a, the lower jaw portion 116 is provided with an irradiation electrode 24b, and the inside of the cutter 15 is provided with an irradiation electrode 24c, and a coaxial cable 40 is connected to the irradiation electrode 24c. Although not shown, the irradiation electrodes 24 (24a, 24b, 24c) arranged on the upper jaw portion 14, the cutter 15 and the lower jaw portion 116 have an insulating layer arranged on the outside, and the upper jaw portion 14, the cutter insulated from 15 and the lower jaw 116.

As shown in the enlarged cross-sectional view of FIG. 3 at (b), the coaxial cable 40 is composed of a central conductor 41, an insulator 42, an outer conductor 43, and an insulating coating 44 with the central conductor 41 interposed therebetween from the center to the outer diameter. They are arranged in this order.

The central conductor 41 is a linear conductor arranged at the center of the coaxial cable 40, and may be a single conductor having an appropriate diameter, or may be composed of a plurality of core wires.

The insulator 42 surrounds an outside of the central conductor 41 is made of resin for insulating the central conductors 41 and the outer 43, and has a cylindrical shape having a predetermined thickness.

The outer conductor 43 is composed of braided wires provided along the outer peripheral surface of the insulator 42.

The insulating coating 44 is a coating having an insulating property and surrounds the outside of the outer conductor 43.

As described above, the coaxial cable 40 in which the central conductor 41, the insulator 42, the outer conductor 43, and the insulating coating 44 are arranged in this order from the center side has appropriate flexibility.

As shown in FIG. 3, the irradiation electrode 24a and the irradiation electrode 24c are connected to the central conductor 41 of the coaxial cable 40, the irradiation electrode 24b is connected to the outer conductor 43, and electromagnetic waves (microwaves) are irradiated from the irradiation electrodes 24a and 24c to the irradiation electrode 24b. Further, the electrode structure may be a coaxial structure as shown in FIGS. 16 and 17, and the polarity of the connection may be a different electrode connection structure as shown in FIG. 20.

One of the first electrode portion of the irradiation electrodes 24a and 24c of the upper jaw portion 14 and the cutter 15 and the second electrode portion of the irradiation electrode 24b provided on the lower jaw portion 116 is connected to the central conductor 41 of the coaxial cable 40. The other will be connected to the outer conductor 43. In this way, the forceps 1 provided with the irradiation electrodes 24a and 24c may irradiate microwaves from one of the first electrode portion of the irradiation electrodes 24a and 24c and the second electrode portion of the irradiation electrode 24b toward the other. As a result, the target portion gripped by the gripping mechanism 17 composed of the lower jaw portion 116 and the upper jaw portion 14 can be coagulated by irradiating the target portion with microwaves. Further, if necessary, the radiation electrode 24c may be electrically connected to the radiation electrode 24b, and a microwave may be irradiated between the upper jaw 14 and the cutter 15 to coagulate the living tissue.

The operation of the forceps 1 in which each element is configured as described above will be described below.

By a user who inserts a finger into the ring portion 113 of the main body frame 11 and the ring portion 131 of the trigger handle 13, the trigger handle 13 is rotated in a direction closer to the fixed handle frame 111 (a direction in which the lower portion of the trigger handle 13 moves to the left in FIG. 1) with the pivot portion 132 pivotally supported by the support shaft 115 as the center of rotation.

When the trigger handle 13 rotates around the pivot portion 132 in a direction approaching the fixed handle frame 111, the upper pivot portion 133 protruding upward from the pivot portion 132 moves toward the distal end side (right side in FIG. 1). When the upper pivot portion 133 moves to the distal end side F, the slide frame 12 that pivotally supports the upper pivot portion 133 with the upper support shaft 121 slides and moves to the distal end side F along the reference frame 112.

When the slide frame 12 moves to the distal end side F, the tip upper support shaft 122 that pivotally supports the rotary bearing portion 142 of the upper jaw portion 14 also moves to the distal end side F. The upper jaw portion 14 in which the lower shafted portion 141 is pivotally supported by the tip support shaft 117 of the main body frame 11 and the rotary bearing portion 142 above it is pivotally supported by the tip upper support shaft 122 is the tip upper support shaft. Since 122 moves to the distal end side F, the distal end side F is pivoted in a direction approaching the lower jaw portion 116 with the distal end support shaft 117 as the center (see FIG. 4).

Further, as described above, in the cutter 15, the lower shafted portion 151 is pivotally supported by the tip support shaft 117 of the main body frame 11, and the upper rotation shaft portion 152 is pivotally supported by the tip upper support shaft 122. Therefore, as the tip upper support shaft 122 moves to the distal end side F, the cutter 15 pivots around the tip support shaft 117 in the direction in which the distal end side F approaches the lower jaw portion 116 together with the upper jaw portion 14 (See Fig. 4).

At this time, since the upper end of the cutter 15 is regulated by the protrusion restricting portion 143 of the upper jaw portion 14, as described above, the cutter 15 is moved as the tip upper support shaft 122 moves to the distal end side F. Although the distal end side F pivots in the direction approaching the lower jaw 116 together with the upper jaw 14 around the tip support shaft 117, even if the pivot of the cutter 15 is delayed from the pivot of the upper jaw 14, the protrusion is restricted. The portion 143 causes the upper jaw portion 14 and the cutter 15 to both pivot.

As a result, in the initial state (open state) as shown in FIG. 6 at (a), the blood vessel B, which is the target portion in the living tissue, is arranged between the upper jaw portion 14 and the lower jaw portion 116, and as shown in FIG. 6 at (b). By sandwiching the blood vessel B between the upper surface of the lower jaw portion 116 and the bottom surface of the upper jaw portion 14, the blood vessel B can be gripped and crashed by the gripping mechanism 17 composed of the lower jaw portion 116 and the upper jaw portion 14. In addition, in order to avoid the problem that the position of the microwave irradiation part and the tissue coagulation is displaced, which may be a problem in the case of a thick blood vessel, an operator grips it with the forceps of the present application, crashes it to perform coagulation, and seal the blood vessel once. By gripping the side of the sealed part again, crashing, coagulating, and cutting, the proximal side of the thick blood vessel is sealed twice, and the peripheral side may be sealed by the blade.

Although the tip upper support shaft 122 is loosely fitted in the rotary bearing portion 142 formed in the elongated hole shape, the upper jaw portion 14 and the cutter 15 are separated from the lower jaw portion 116 by the spring 16. Since it is urged, the upper tip support shaft 122 does not move in the rotary bearing portion 142, and the upper jaw portion 14 and the cutter 15 rotate as described above.

At this time, the click convex portion 118 provided on the upper surface of the reference frame 112 and the click convex portion 123 provided on the bottom surface of the slide frame 12 are separated from each other in the longitudinal direction L in the initial state (open state). As shown in the enlarged view of the portion A in FIG. 4 at (b), the slide frame 12 slides toward the distal end side F with respect to the reference frame 112, so that the slide frame 12 approaches and abuts or contact in the longitudinal direction L.

As described above, the trigger handle 13 is operated to grip the target portion such as the blood vessel B in the state shown in FIG. 4 and FIG. 6 at (b) in which the upper jaw portion 14 is close to the lower jaw portion 116 by the gripping mechanism 17 which is called a gripping state.

When the trigger handle 13 is further operated in the direction closer to the fixed handle frame 111 from this gripping state, the slide frame 12 is further moved to the distal end side F with respect to the reference frame 112 (to crash).

Further, when the slide frame 12 moves to the distal end side F, the cutter 15 is pivoted by the cutting blade 153 over the upper surface of the lower jaw portion 116 with the tip support shaft 117 as the center, as shown in FIG. 5 at (b).

As a result, as shown in FIG. 6 at (c), the cutter 15 moves along the side surface of the upper jaw portion 14, and the blood vessel B gripped by the gripping mechanism 17 can be cut by the cutting blade 153 of the cutter 15.

As described above, by further operating the trigger handle 13, as shown in FIG. 5 at (b), the cutting state in which the cutter 15 is rotated so that the cutting blade 153 exceeds the upper surface of the lower jaw portion 116 is in the cutting state.

However, the upper jaw portion 14 is in contact with the upper surface of the lower jaw portion 116, and cannot rotate even if the slide frame 12 further slides. By that amount, the tip upper support shaft 122 moves in the elongated hole-shaped rotary bearing portion 142, and the slide frame 12 moves relative to the upper jaw portion 14.

As described above, the cutter 15 rotates further due to the further movement of the slide frame 12, but the support shaft 122 on the tip of the rotary bearing portion 142 moves and the upper jaw portion 14 does not rotate, that is, the length. The upper jaw portion 14 and the cutter 15 are differentiated in the rotational direction around the tip support shaft 117 by a differential mechanism in which the tip upper support shaft 122 moves on the hole-shaped rotary bearing portion 142. The spring 16 is deformed in the contracting direction due to the differential between the upper jaw portion 14 and the cutter 15. Therefore, when the force for operating the trigger handle 13 is released, the compression of the compression coil spring 13a shown in FIG. 1 is released, the slide frame 12 moves to the proximal end side R, and the urging force of the spring 16 opens the cutter 15. Along with the movement in the direction, the upper jaw portion 14, that is, both members 14 and 15, cooperate to automatically return to the open state of FIG. 1.

Even if the pivot of the cutter 15 in the opening direction is faster than the pivot of the upper jaw 14 in the opening direction due to the urging force of the spring 16, the upper end of the cutter 15 is regulated by the protrusion restricting portion 143. Therefore, both the upper jaw portion 14 and the cutter 15 are pivoted in the opening direction, and it is possible to prevent the upper portion of the cutter 15 from protruding upward from the upper surface of the upper jaw portion 14.

Further, in this cut state, the click convex portion 118 and the click convex portion 123 that are in contact with each other in the above-mentioned gripping state are as shown in the enlarged view of the A portion in FIG. 5 at (b). While the click convex portion 118 and the click convex portion 123 are bent, one of them gets over the other, so that a click feeling is generated. Since this click feeling is transmitted to the user via the main body frame 11 and the trigger handle 13, the user recognizes that the disconnected state has been reached, that is, in a series of operations of the trigger handle 13, the gripped state is changed to the disconnected state. The clicked protrusions 118 and 123 can notify the user.

When the force for operating the trigger handle 13 is released and the slide frame 12 moves to the base end side R and returns to the initial state (open state), the inclination angle exceeds the gentle surface side, so that the slope is steep. There is no click feeling like when the sloped surfaces get over each other.

As described above, the forceps 1 performs gripping of the target portion such as the blood vessel B by the upper jaw portion 14 and the lower jaw portion 116 constituting the gripping mechanism 17, and cutting of the target portion by the cutter 15 in the gripping/crashing state by a series of operations by the handle 13. Then, during the gripping, crashing, cutting of the target portion by a series of operations of the trigger handle 13, the target portion can be coagulated by irradiating the target portion with a microwave from the irradiation electrode 24.

Further, since the differential mechanism is provided by the elongated hole-shaped rotary bearing portion 142 that loosely fits the support shaft 122 on the tip, in a series of operations of the trigger handle 13, after gripping by the gripping mechanism 17, the target portion may be cut by the cutter 15.Further, it is possible to provide a notification unit for notifying the user by the click convex portions 118 and 123 of the transition from the gripping state to the cutting state in the series of operations of the trigger handle 13.

The notification unit may have a structure in which a moving sensor is attached instead of the click protrusions 118 and 123 to notify the user by an electronic signal or an optical signal. According to this embodiment, it is possible to provide a multifunctional forceps for gripping, coagulating, or cutting alone or in combination.

Subsequently, the forceps 1X of a second embodiment of the present invention are shown in FIGS. 7 to 11.

FIG. 7 shows a front view of the forceps 1X of this embodiment. FIG. 8 shows an explanatory diagram of the forceps 1X of FIG. 8 at (a) shows a rear view of the forceps 1X, and FIG. 8 at (b) shows a side view of the forceps 1X on the cutting mechanism side.

FIG. 9 shows a rear view of the forceps 1X of FIG. 8, and a partial cross-sectional view taken along the line E-E in FIG. 8 at (b) .

FIG. 10 shows an explanatory view of the forceps 1X in the gripped state. FIG. 10 at (a) shows a front view of the forceps 1X in the gripped state, FIG. 10 at (b) shows a rear view of the forceps 1X in the same state, and FIG. 10 at (c) shows a cutting mechanism side of the forceps 1X in the same state. A side view is shown, and FIG. 10 at (d) shows a partial cross-sectional view taken along the line F-F in FIG. 10 at (c).

FIG. 11 shows an explanatory diagram of the forceps 1X in the cut state. 11 at (a) shows a front view of the forceps 1X in the cut state, FIG. 11 at (b) shows a rear view of the forceps 1X in the same state, and FIG. 11 at (c) shows the cutting mechanism side of the forceps 1X in the same state. A side view is shown, and FIG. 11 at (d) shows a partial cross-sectional view taken along the line G-G in FIG. 11 at (c).

In the following description of the forceps 1X, the same components as those of the forceps 1 are designated by the same reference numerals, and detailed description thereof will be omitted.

The forceps 1X includes a main body frame 11X, a first slide plate 12Xa, a second slide plate 12Xb, a trigger handle 13X, an upper jaw portion 14, a cutter 15, and a coil spring 16X.

In the forceps 1X, the first slide plate 12Xa and the second slide plate 12Xb slide with respect to the reference frame 112 with respect to the forceps 1 in which the slide frame 12 slides on the upper surface of the reference frame 112 of the main body frame 11 in the longitudinal direction L. Then, the upper jaw portion 14 and the cutter 15 are configured to rotate.

. More specifically based on FIG. 7, the main body frame 11X is composed of the fixed handle frame 111 and the reference frame 112X that intersect at an obtuse angle, similarly to the main body frame 11 composed of the fixed handle frame 111 and the reference frame 112 of FIG. 1.

As shown in FIG. 10 at (a) and (b), with respect to the reference frame 112 in which the slide frame 12 of FIG. 1 slides along the upper surface, the reference frame 112X has a slide groove (not shown), on which the first slide plate 12Xa and the second slide plate 12Xb slide, formed in the upper half portion on the back surface side.

The first slide plate 12Xa and the second slide plate 12Xb are formed in a plate shape, are laminated in the slide groove provided in the reference frame 112X in the thickness direction, and independently slidably stored.

As shown in FIG. 8, the base end side R of the first slide plate 12Xa is provided with a middle support shaft 121Xa that pivotally supports the upper pivot portion 133 provided on the trigger handle 13X, and the distal end side F is provided with a tip upper support shaft 122Xa that pivotally supports the cutter 15.

As shown in FIG. 9, on the distal end side F of the second slide plate 12Xb, a tip upper support shaft 122Xb for supporting the cutter 15 is provided.

In addition, as shown in FIG. 11, a first base end side R is provided with a contact ring portion 124Xb through which the coil spring 16X abuts and the insertion shaft 161X through which the inside of the coil spring 16X is inserted.

The second slide plate 12Xb configured in this way is formed to have a length that is about the length of the coil spring 16X, which will be described later, and a length that the proximal end side R is shorter than that of the first slide plate 12Xa.

Further, the first slide plate 12Xa and the second slide plate 12Xb which are laminated and arranged in the thickness direction in the slide groove provided in the reference frame 112X are arranged so that the first slide plate 12Xa is on the back side. Therefore, the second slide plate 12Xb is sandwiched between the reference frame 112X and the first slide plate 12Xa in the front-back direction.

The first slide plate 12Xa and the second slide plate 12Xb are provided with a long hole-shaped regulation hole 123X long in the longitudinal direction L into which the slide frame 112Xa provided in the slide groove of the reference frame 112X is loosely fitted.

The trigger handle 13X is provided in addition to the axis portion 132 and the axis portion 133, as compared with the axis portion 132 and the trigger handle 13 (FIG. 1) in which the axis portion 133 is provided on the upper end side of the axis portion 132 with an upper stage pivot portion 134 provided above the upper axis portion 133. Further, the upper stage pivot portion 134 of the trigger handle 13X is rotatably provided with a pressing block 135X having an insertion shaft 161X. The compression coil spring 13a described with reference to FIG. 1 may be provided between the fixed handle frame 111 of the main body frame 11X and the trigger handle 13X. The trigger handle 13X is in addition to the axis portion 132 and the axis portion 133, as compared with the axis portion 132 and the trigger handle 13 (see FIG. 1) in which the axis portion 133 is provided on the upper end side of the axis portion 132. The upper pivot portion 134 is provided above the upper axis portion 133. Further, the upper stage pivot portion 134 of the trigger handle 13X is rotatably provided with a pressing block 135X having an insertion shaft 161X. The compression coil spring 13a described with reference to FIG. 1 may be provided between the fixed handle frame 111 of the main body frame 11X and the trigger handle 13X.

The insertion shaft 161X is inserted through the inside of the coil spring 16X and is inserted into the contact ring portion 124Xb. A flange portion 162X having a diameter larger than that of the abutting ring portion 124Xb is provided at the end portion of the distal end side F of the insertion shaft 161X.

When the trigger handle 13X is operated, the forceps 1X having each element configured in this way moves the upper pivot portion 133X and the upper stage pivot portion 134 to the distal end side F with the pivot portion 132 as the rotation axis.

When the upper pivot portion 133 moves to the distal end side F, the first slide plate 12Xa having the middle stage support shaft 121 that pivotally supports the upper pivot portion 133 slides to the distal end side F with respect to the reference frame 112X.

As shown in FIG. 10, when the first slide plate 12Xa moves to the distal end side F, the cutter 15 that pivotally supports the rotation shaft portion 152 with the tip upper support shaft 122Xa is pivotally supported by the tip support shaft 117. It rotates along the side surface of the upper jaw portion 14 with the portion 151 as an axis.

On the other hand, when the upper stage pivot portion moves to the distal end side F, the pressing block 135X rotatably provided on the upper stage pivot portion also moves to the distal end side F.

When the pressing block 135X moves, the coil spring 16X through which the insertion shaft 161X is inserted is pressed against the distal end side F. Since the distal end side F of the coil spring 16X pressed against the distal end side F by the pressing block 135X is in contact with the contact ring portion 124Xb, the contact ring portion 124Xb is pressed against the distal end side F and the second slide plate 12Xb is moved.

When the second slide plate 12Xb moves to the distal end side F, the lower shafted portion 141 is pivotally supported by the tip support shaft 117 of the main body frame 11X, and the rotary bearing portion 142 above it is pivotally supported by the tip upper support shaft 122Xb. In the upper jaw portion 14, the tip upper support shaft 122Xb moves to the distal end side F. As a result, the distal end side F pivots in the direction approaching the lower jaw portion 116 around the tip support shaft 117, and the target portion such as the blood vessel B is gripped by the gripping mechanism 17 composed of the lower jaw portion 116 and the upper jaw portion 14 that is in a gripping state (see FIG. 10).

In the first slide plate 12Xa and the second slide plate 12Xb that move to the tip (distal end) side F by operating the trigger handle 13X, the slide amount of the second slide plate 12Xb that pivotally supports the upper stage pivot portion 134 whose distance from the pivot portion 132 is longer than that of the first slide plate 12Xa that pivotally supports the upper pivot portion 133.

When the trigger handle 13X is further operated in the direction closer to the fixed handle frame 111 from this gripped state, the first slide plate 12Xa is further moved to the tip side F with respect to the reference frame 112X.

Further, when the first slide plate 12Xa moves to the tip side F, as shown in the enlarged view of the portion B in FIG. 11 at (b), the cutting blade 153 of the cutter 15 centering on the tip support shaft 117 pivots beyond the upper surface of the lower jaw portion 116.

As a result, the cutter 15 moves along the upper jaw portion 14, and the blood vessel B gripped by the gripping mechanism 17 becomes in a cutting state in which the blood vessel B may be cut by the cutting blade 153 of the cutter 15.

However, the upper jaw portion 14 is in contact with the upper surface of the lower jaw portion 116, and cannot rotate even if the second slide plate 12Xb further slides.

When the trigger handle 13X is further operated in the direction closer to the fixed handle frame 111, the upper stage pivot portion also moves to the tip (distal end) side F, and the pressing block 135X also moves to the tip side F together with the upper stage pivot portion. However, as described above, since the upper jaw portion 14 cannot be further rotated, the second slide plate 12Xb also cannot slide.

Therefore, the coil spring 16X contracts and comes close to the contact ring portion 124Xb of the second slide plate 12Xb that cannot slide and the pressing block 135X.That is, when the coil spring 16X contracts, the contact ring portion 124Xb and the pressing block 135X are differentiated, and the trigger handle 13X is operated in a direction further closer to the fixed handle frame 111, so that the cutter 15 rotates. However, the upper jaw portion 14 may generate a differential that does not rotate.

As described above, the coil spring 16X is deformed in the contracting direction in the cutting state in which the cutter 15 and the upper jaw portion 14 are differentially operated by the further operation of the trigger handle 13X.Therefore, when the force for operating the trigger handle 13X is released, the trigger handle 13X moves in the expanding direction via the pressing block 135X due to the urging force of the coil spring 16X.

The facing surfaces of the first slide plate 12Xa and the second slide plate 12Xb may be provided with the same configuration as the click convex portion 118 and the click convex portion 123 in the forceps 1 of FIG. 2. Even in this case, one of the click feeling generated when overcoming the other makes the user to recognize cutting state.

As described above, the forceps 1X of this embodiment also has the same gripping state as the forceps 1 of FIG. 1. Gripping the target portion such as blood vessel B by the upper jaw portion 14 and the lower jaw portion 116 constituting the grip mechanism 17 and cutting the target portion by the cutter 15 in the gripped state may be performed by a series of operations by the trigger handle 13X. Then, during the gripping, crashing, and cutting of the target portion by a series of operations of the trigger handle 13X, the target portion can be coagulated by irradiating the target portion with a microwave from the irradiation electrode 24.

Further, since the coil spring 16X is provided with a differential mechanism by contracting, the target portion can be cut by the cutter 15 after gripping and crashing by the gripping mechanism 17 in a series of operations of the trigger handle 13X.

The upper jaw portion 14 of the forceps 1X may be provided with a protrusion restricting portion (see 143 of FIG. 3 at (a)) that restricts the cutter 15 from protruding from the upper surface of the upper jaw portion 14. As a result, the cutter 15 does not protrude from the upper surface of the upper jaw portion 14, and can be used safely.

Subsequently, the forceps 1Y of another embodiment will be described with reference to FIGS. 12 to 15.

FIG. 12 shows a front view of the forceps 1Y of this embodiment, and FIG. 13 shows a rear view of the forceps 1Y of FIG 12.

FIG. 14 shows an explanatory view of the forceps 1Y in a gripping state. FIG. 14 at (a) shows a front view of the forceps 1Y in the gripping state, and FIG. 14 at (b) shows a rear view of the forceps 1Y in the same state.

FIG. 15 shows an explanatory diagram of the forceps 1Y in a cutting state. FIG. 15 at (a) shows a front view of the forceps 1Y in the cutting state, and FIG. 15 at (b) shows a rear view of the forceps 1Y in the same state.

In the following description of forceps 1Y, the same components as those of forceps 1 in FIG. 1 and forceps 1X in FIG. 7 are designated by the same reference numerals, and detailed description thereof will be omitted.

In FIG. 12, the forceps 1Y includes a main body frame 11Y, a first slide plate 12Ya (see FIG. 13), a second slide plate 12Yb, a trigger handle 13Y, an upper jaw portion 14, and a slide cutter 15Y (see FIG. 13). And a coil spring 16Y

With respect to the forceps 1 in which the slide frame 12 slides in the longitudinal direction L on the upper surface of the reference frame 112 of the main body frame 11 shown in FIG. 1, the forceps 1Y has the upper jaw in which the second slide plate 12Yb slides with respect to the reference frame 112Y. The portion 14 rotates, and the slide cutter 15Y is configured to slide and cut along with the slide of the first slide plate 12Y provided at the end portion of the distal end side F.

More specifically, the main body frame 11Y is composed of the fixed handle frame 111 and the reference frame 112Y that intersect at an obtuse angle, similarly to the main body frame 11 composed of the fixed handle frame 111 and the reference frame 112 shown in FIG. 1.

Similar to the reference frame 112 in which the slide frame 12 shown in FIG. 1 slides along the upper surface, the first slide plate 12Ya and the second slide plate 12Yb are configured to slide along the upper surface of the reference frame 112Y. The reference frame 112Y is provided with a regulation frame 124thatregulates the first slide plate 12Ya and the second slide plate 12Yb that slide along the upper surface so as to be slidable.

The first slide plate 12Ya and the second slide plate 12Yb are formed in a plate shape, are laminated in the thickness direction, and are independently slidable along the upper surface of the reference frame 112Y and are regulated by the regulation frame 124.

In FIG. 13, a slide cutter 15Y is provided at the end of the distal end side F of the first slide plate 12Ya, and an upper support shaft 121Ya for pivotally supporting the drive shaft 181Y of the arm 18Y described later is provided at the intermediate portion.

Returning to FIG. 12, an upper support shaft 121Yb that pivotally supports the drive shaft 181Y of the arm 18Y, which will be described later, is provided in the middle portion of the second slide plate 12Yb to which the upper jaw portion 14 is connected to the distal end.

The upper support shaft 121Yb has a long hole shape long in the longitudinal direction L, and the drive shaft 181Y can be pivotally supported in the longitudinal direction L. Further, inside the upper support shaft 121Yb, a coil spring 16Y that urges the drive shaft 181Y to the base end side R (left side in FIG. 12) is arranged on the distal end side F of the drive shaft 181Y.

The trigger handle 13Y is provided with an upper pivot portion 133 above the pivot portion 132 pivotally supported by the reference frame 112Y so as to project upward from the first slide plate 12Ya and the second slide plate 12Yb.

An arm 18Y tilting downward and on the tip side F is connected to the upper pivot portion 133, and the above-mentioned drive shaft 181Y is provided at the tip of the arm 18Y.

When the trigger handle 13Y is operated, the forceps 1Y having each element configured in this way moves the upper pivot portion 133 to the distal end side F with the pivot portion 132 as a rotation axis.

When the upper pivot portion 133 moves to the tip side F, the arm 18Y connected to the upper pivot portion 133 also moves to the tip side F while changing the inclination angle. When the arm 18Y moves to the distal end side F while the inclination angle changes, the first slide plate 12Ya (FIG. 13) that pivotally supports the drive shaft 181Y of the arm 18Y with the upper support shaft 121Y slides to the distal end side F with respect to the reference frame 112Y.

As shown in the enlarged view of the portion A in FIG. 14 at (a), when the first slide plate 12Ya moves to the distal end side F, the slide cutter 15Y provided on the distal end side F of the first slide plate 12Ya also moves to the distal end side F along the upper surface of the lower jaw portion 116 in the reference frame 112Y. However, the cutting blade 153Y of the slide cutter 15Y moving to the distal end side F is moved to the extent that it does not overlap the middle portion of the upper jaw portion 14.

Further, as shown in the enlarged view of the portion B in FIG. 14 at (b), the arm 18Y moves to the distal end side F while the inclination angle changes as described above with the movement of the upper pivot portion 133 to the distal end side F. When it moves, the drive shaft 181Y is loosely fitted to the upper support shaft 121Yb and is urged to the proximal end side R along the coil spring 16Y, so that the second slide plate 12Yb also moves to the distal end side F.

More specifically, in FIG. 12, when the second slide plate 12Yb moves to the distal end side F, similar to the operation as in FIG. 1, the lower shafted portion 141 is pivotally supported by the distal end support shaft 117 of the main body frame 11Y, and regarding the upper jaw portion 14 whose upper rotary bearing portion 142 is pivotally supported by the distal end upper support shaft 122Yb, the distal end upper support shaft 122Yb moves to the distal end side F. As a result, the distal end side F is pivotally moved in the direction approaching the mandibular portion 116 around the distal end support shaft 117 as the center, and as shown in FIG. 14 at (b), the gripping mechanism 17 composed of the lower jaw portion 116 and the upper jaw portion 14 brings the target portion such as blood vessel B in a gripping state where the target portion may be gripped.

When the trigger handle 13Y is further operated in the direction closer to the fixed handle frame 111 from this gripped state, the first slide plate 12Ya moves further to the distal end side F with respect to the reference frame 112.

As shown in FIG. 15 at (a), when the first slide plate 12Ya is further moved to the distal end side F, the slide cutter 15Y provided at the distal end of the first slide plate 12Ya moves around the distal end support shaft 117, and the blade 153Y moves to the vicinity of the distal end surface of the upper jaw portion 14.

Thus, the slide cutter 15Y moves along the reference frame 112Y, and the vessel B gripped by the gripping mechanism 17 is cut by the cutting blade 153Y of the slide cutter 15Y.

However, the upper jaw portion 14 is in contact with the upper surface of the lower jaw portion 116, and cannot rotate even if the second slide plate 12Yb further slides.

When the trigger handle 13Y is further operated in a direction closer to the fixed handle frame 111,the drive shaft 181Y of the arm 18Y pivotally supported by the upper pivot portion 133also moves to the distal end side F. However, as described above, the second slide plate 12Yb cannot slide to the distal end side F. Therefore, the drive shaft 181Y presses the coil spring 16Y toward the distal end side F to contract the drive shaft 181Y against the urging force of the coil spring 16Y that urges the drive shaft 181Y to the proximal end side R. That is, as shown in FIG. 15 at (b), which is an enlarged view of the portion A in FIG. 15 at (a), the trigger handle 13Y is operated in a direction further closer to the fixed handle frame 111, and the coil spring 16Y contracts to slide. The cutter 15Y slides to the distal end side F, but the upper jaw portion 14 may generate a differential operation that it does not rotate.

As described above, the coil spring 16Y is deformed in the contracting direction in the cutting state in which the slide cutter 15Y and the upper jaw portion 14 are differentially operated by the further operation of the trigger handle 13YTherefore, when the force for operating the trigger handle 13Y is released, the trigger handle 13Y moves in the expanding direction via the arm 18Y due to the urging force of the compression coil spring 13a.

The facing surfaces of the first slide plate 12Ya and the second slide plate 12Yb may be provided with the same configuration as the click convex portion 118 and the click convex portion 123 in the forceps 1 of FIG. 4 at (b) described above. Even in this case, the user can be made to recognize that the user has been disconnected by the click feeling generated when one gets over the other.

As described above, the forceps 1Y also grips the target portion such as the blood vessel B by the upper jaw portion 14 and the lower jaw portion 116 constituting the gripping mechanism 17 and cuts the target portion of the gripped state by the slide cutter 15Y which may be performed by a series of operations by the trigger handle 13, similarly to the forceps 1 in FIG. 1 and the forceps 1X in FIG. 7. Then, during the gripping / crashing / cutting of the target portion by a series of operations of the trigger handle 13, the target portion can be coagulated by irradiating the target portion with a microwave from the irradiation electrode 24.

Further, since the coil spring 16Y is provided with a differential mechanism by contracting, the target portion may be cut by the slide cutter 15Y after being gripped by the gripping mechanism 17 in a series of operations of the trigger handle 13.

Next, FIGS. 16 and 17 show an end effector 10a for microwave irradiation on a living tissue as another embodiment of the present invention. The forceps 1, 1X, and 1Y of the above-described embodiments all include an irradiation electrode 24 for irradiating an electromagnetic wave, and a specific electrode structure will be described in this embodiment.

FIG. 16 shows a schematic front view of the end effector 10a of this embodiment.

FIG. 17 shows a schematic explanatory view of the end effector 10a of FIG. 16. FIG. 17 at (a) shows a side by view taken along the line H-H in FIG. 16, FIG. 17 at (b) shows a partial vertical sectional view of the end effector 10a, and FIG. 17 at (c) shows an enlarged plane view of electrodes by view taken along the line I-I in FIG. 16.

The end effector 10a is used for a scissors-type multifunctional surgical instrument, and corresponds to the cutting mechanism 10 in the above-mentioned forceps 1. Therefore, the same components as those in the forceps 1 of the embodiment of FIG. 1 described above are designated by the same reference numerals. Further, the end effector in this embodiment may constitute the above-mentioned cutting device, cutting mechanism, and forceps, and includes other similar configurations.

In addition, in FIGS. 16 and 17, a part of the distal end side F of the end effector 10a is shown.

The end effector 10a has a reference shaft 11a (corresponding to the reference frame 112 in the cutting mechanism 10) that penetrates the bellows-shaped flexible portion 101 provided at the distal end of the tubular support 100 attached to the scissors-type multifunctional surgical instrument, and a movable frame 12a (corresponding to the slide frame 12 in the cutting mechanism 10) provided with an upper jaw portion 14a (corresponding to the upper jaw portion 14 in the cutting mechanism 10) and a cutter 15a (corresponding to the cutter 15 in the cutting mechanism 10) at the distal end.

In the reference shaft 11a and the movable frame 12a, the portion corresponding to the inside of the flexible portion 101 is configured to have flexibility that may be deformed following the movable flexible portion 101. However, a wire may be used as a drive.

The operation of the upper jaw portion 14a and the cutter 15a in the end effector 10a configured in this way is the same as that of the upper jaw portion 14 and the cutter 15 of the cutting mechanism 10 in the forceps 1 described above, by moving the frame 12a in the longitudinal direction L with respect to the reference axis 11a, the target portion can be gripped and crashed by the gripping mechanism 17a composed of the lower jaw portion 116a and the upper jaw portion 14a provided at the end of the distal end side F of the reference shaft 11a, and can be cut with the cutter 15.

In addition, a distal end side on the back side of the upper jaw portion 14a is provided with a protrusion restricting portion 43 that projects to the back side and restricts the cutting cutter 15a that rotates along the side surface of the upper jaw portion 14a on the back surface side to protrude upward from the upper surface of the upper jaw portion 14a. As a result, even if the pivot of the cutter 15a in the opening direction is faster than the pivot of the upper jaw 14a in an opening direction, the upper end of the cutter 15a is regulated by the protrusion restricting portion 143, and both the upper jaw portion 14a and the cutter 15a are pivoted in the opening direction, and it is possible to prevent the upper portion of the cutter 15a from protruding upward from the upper surface of the upper jaw portion 14a.

A coaxial electrode 20 along the longitudinal direction L is provided on the upper surface of the lower jaw portion 116a and the bottom surface of the upper jaw portion 14a of the end effector 10a configured in this way.

Further, in the end effector 10a, coaxial cable 40 is connected with microwave generator 30 and the coaxial electrode 20.The microwave generator 30 may be provided inside the end effector 10a.

As shown in the enlarged cross-sectional view of the portion A in FIG. 16, the coaxial cable 40 is composed of an insulator 42, an outer conductor 43, and an insulating coating 44 with the central conductor 41 and the central conductor 41 interposed therebetween, from the center to the outer diameter. They are arranged in this order and have appropriate flexibility.

As shown in the enlarged view of the A portion in FIG. 17 at (a), the coaxial electrodes 20 provided on the bottom surface of the upper jaw portion 14a and the upper surface of the lower jaw portion 116a have central conductors 21 and semicircular insulators 22 having semicircular cross sections, and semicircular tubular conductors 23 arranged outside the semicircular insulators 22.

As shown in FIG. 17 at (a), the thus configured coaxial electrodes 20 have semi-cylindrical and a predetermined length, and as shown in FIG. 17 at (c), on a bottom surface of the upper jaw portion 14a and an upper surface of the lower jaw portion 116a, are arranged along the longitudinal direction so as to face the flat surfaces each other. Although not shown, the coaxial electrodes 20 arranged on the upper jaw portion 14a and the lower jaw portion 116a have insulating layers arranged on the outside and are insulated from the upper jaw portion 14a and the lower jaw portion 116a.

Then, as shown in FIG. 17 at (b), the central conductor 21 of each coaxial electrode 20 provided in the upper jaw portion 14a and the lower jaw portion 116a is connected to the central conductor 41 of the coaxial cable 40, and the half of the coaxial electrode 20 is connected. The circular conductor 23 and the outer conductor 43 of the coaxial cable 40 are connected to form the same pole connection. If necessary, one of them may be connected in the opposite polarity as shown in FIG. 20.

In this way, the coaxial electrode 20 connected to the microwave generator 30 via the coaxial cable 40 may irradiate microwaves between the central conductor 21 of the coaxial electrode 20 and the semicircular conductor 23 via the coaxial cable 40 when the microwave generator 30 activates.

When microwaves are irradiated from the coaxial electrode 20 in a state where the blood vessel B, which is the target portion, is gripped and crashed by the gripping mechanism 17a, the blood vessel B gripped and crashed by the gripping mechanism 17a is coagulated by the microwave. Then, in the blood vessel B, the portion solidified by the microwave irradiated from the coaxial electrode 20 may be cut by the cutter 15a.As a modification of this embodiment, FIG. 16 shows an embodiment in which the electronic module 31 is built in the end effector 10a.

The above-described microwave generator 30 is an example which is incorporated in the end effector 10a, but it may incorporate a microwave irradiation module 222 of FIG. 25, or incorporate appropriate electronic circuitry of the surgical device 221 shown in FIG. 27 (b). By providing the electronic module 31 in the end effector 10a, the medical device may be miniaturized and the convenience of surgery is enhanced.

Subsequently, FIGS. 18 and 19 show an end effector 10b as another embodiment of the present invention.

FIG. 18 shows a schematic explanatory view of the end effector 10b of this embodiment. FIG. 18 at (a) shows a schematic front view of the end effector 10b in a normal state, and FIG. 18 at (b) shows a schematic front view of the end effector 10b in a cut state.

FIG. 19 shows a schematic explanatory view of the end effector 10b. FIG. 19 at (a) shows a schematic cross-sectional view of a cutting mechanism in the end effector 10b in a normal state, FIG. 19 at (b) shows a schematic cross-sectional view of the end effector 10b in a cut state, and FIG. 19 at (c) shows a coaxial electrode. A schematic cross-sectional view of a cutting mechanism with different arrangements is shown.

In FIG. 16, with respect to the end effector 10a in which the gripping mechanism 17a composed of the lower jaw portion 116a and the upper jaw portion 14a is further provided with a cutter 15a, and the target portion gripped by the gripping mechanism 17a is cut by the cutter 15a, the end effector 10b of FIG. 18 is provided with a fixed blade 119b instead of the lower jaw portion 116a at the distal end of the reference shaft 11b, and provided with a movable blade 14b instead of the upper jaw portion 14a to form a cutting mechanism 19.

In the end effector 10b configured in this way, the movable frame 12b is moved to the distal end side F with respect to the reference shaft 11b, so that the movable blade 14b connected to the movable frame 12b supports the distal end provided on the reference shaft 11b. It rotates around the shafted portion 141b pivotally supported by the shaft 117b in a direction close to the fixed blade 119b. Then, as shown in FIG. 18 at (b) and FIG. 19 at (b), the fixed blade 119b and the movable blade 14b are closed, and the target portion may be cut by the cutting blades 153b and 153a provided respectively.

Coaxial electrodes 20 are provided on the side surfaces of the end effector 10b configured as described above on the opposite side of the fixed blade 119b and the movable blade 14b. Then, as shown in FIG. 19 at (a), the central conductor 21 of each coaxial electrode 20 provided on the side surfaces of the fixed blade 119b and the movable blade 14b and the central conductor 41 of the coaxial cable 40 are electrically connected to be coaxial. The semi-circular conductor 23 of the electrode 20 and the outer conductor 43 of the coaxial cable 40 are electrically connected.

Therefore, in the cutting mechanism 19 of the end effector 10b, microwaves may be irradiated from the coaxial electrodes 20 provided on the side surfaces of the fixed blade 119b and the movable blade 14b.

In the end effector 10b configured in this way, microwaves are irradiated from each of the fixed blade 119b and the movable blade 14b in a state where the blood vessel B, which is the target portion, is arranged between the fixed blade 119b and the movable blade 14b. As a result, the blood vessel B arranged between the fixed blade 119b and the movable blade 14b is coagulated. Then, the coagulated portion in the blood vessel B may be cut by the cutting mechanism 19 by operating the movable blade 14b.

Although the blood vessel B may be efficiently coagulated and bound by abutting the blood vessel B on the movable blade 14b and the reference shaft 11b and irradiating the blood vessel B with microwaves from the coaxial electrode 20, the movable blade 14b and the fixed blade 119b and blood vessel B may be slightly separated.

Further, in the end effector 10b, as shown in FIG. 19 at (a) and (b), the coaxial electrodes 20 are provided on the opposite side surfaces of the fixed blade 119b and the movable blade 14b, but as shown in FIG. 19 at (c), the coaxial electrode 20 may be arranged on the outer side surface which is the side separated from the fixed blade 119b and the movable blade 14b.

Further, in the end effector 10b, as described above, the central conductor 21 of the coaxial electrode 20 and the central conductor 41 of the coaxial cable 40 provided on the side surfaces of the fixed blade 119b and the movable blade 14b are electrically connected to each other. The semi-circular conductor 23 of the coaxial electrode 20 and the outer conductor 43 of the coaxial cable 40 are electrically connected.

On the other hand, as shown in FIG. 20 at (a), one central conductor 21 of the coaxial electrode 20 provided on each of the side surfaces of the fixed blade 119b and the movable blade 14b and the outer conductor 43 of the coaxial cable 40 are electrically connected. It may be connected and the semicircular conductor 23 of the coaxial electrode 20 and the central conductor 41 of the coaxial cable 40 may be electrically connected. In this case, the polarities of the coaxial electrodes 20 provided on the side surfaces of the fixed blade 119b and the movable blade 14b are opposite to each other.

Also in this case, when the microwave generator 30 is operated, microwaves may be irradiated between the central conductor 21 and the semicircular conductor 23 at the coaxial electrodes 20 provided on the side surfaces of the fixed blade 119b and the movable blade 14b, respectively. At the same time, the central conductor 21 of one coaxial electrode 20 and the other central conductor 21 having opposite polarities, and the semicircular conductor 23 of one coaxial electrode 20 and the semicircular conductor 23 of the other coaxial electrode 20 Microwaves will be emitted even in the meantime.

Further, as shown in FIG. 20 at (b), the end effector 10a shown in FIG. 17 at (b) may be connected and configured to have coaxial electrodes 20 provided on the facing surfaces of the lower jaw portion 116a and the upper jaw portion 14a having opposite polarities.

Further, although not shown, in the end effector 10a, a coaxial electrode 20 may be provided on the side surface of the cutter 15a in addition to the facing surface between the lower jaw portion 116a and the upper jaw portion 14a, or the coaxial electrode 20 may be provided on the upper jaw portion 14a. The coaxial electrode 20 may be provided on the upper surface of the lower jaw portion 116a and the side surface of the cutter 15a without providing the 20. Further, as shown in FIG. 24 at (f) to (h), the coaxial electrode 20 may be provided only on one of the facing surfaces of the upper jaw portion 14a and the lower jaw portion 116a.

Further, as shown in FIG. 21, instead of the coaxial electrodes 20 provided on the upper jaw portion 14 and the lower jaw portion 116a of the end effector 10a, the above-mentioned irradiation electrodes 24a and 24b may be provided. Further, an irradiation electrode 24c may be provided on the side surface of the cutter 15.FIG. 21 at (a) shows a vertical cross-sectional view of the end effector 10a provided with the irradiation electrode 24 (24a, 24b, 24c), and FIG. 21at (b) shows a partially enlarged vertical cross-sectional view of the end effector 10a provided with the irradiation electrode 24.

Furthermore, as shown in FIG. 22, at least one of the upper jaw portion 14, the cutter 15 and the lower jaw portion 116 in the above-mentioned forceps 1, 1X, 1Y may be provided with a coaxial electrode 20 connected to the microwave generator 30 by a coaxial cable 40.

FIG. 22 at (a) shows a front view of the forceps 1 in which the coaxial electrodes 20 are provided on the upper jaw portion 14 and the lower jaw portion 116. FIG. 22 at (b) shows a front view of the forceps 1X having the coaxial electrodes 20 provided on the upper jaw portion 14 and the lower jaw portion 116, and FIG. 22 at (c) shows a front view of the forceps 1Y provided with the coaxial electrodes 20 on the upper jaw portion 14 and the lower jaw portion 116. As described above, the forceps 1, 1X and 1Y provided with the coaxial electrode 20 in the gripping mechanism 17 composed of the upper jaw portion 14 and the lower jaw portion 116 operate the trigger handle 13 with respect to the target portion gripped by the gripping mechanism 17. After irradiating microwaves from both coaxial electrodes 20 to coagulate, the target portion may be cut with the cutters 15 and 15Y

In FIG. 22, both the upper jaw portion 14 and the lower jaw portion 116 constituting the gripping mechanism 17 are provided with the coaxial electrode 20, but one of the upper jaw portion 14 and the lower jaw portion 116 may be provided with the coaxial electrode 20. The cutters 15 and 15Y may be provided with the coaxial electrode 20.

Furthermore, as shown in FIG. 23, depending on the specifications as a cutting device, a coaxial electrode 20 and an irradiation electrode 24 do not have to be provided on the upper jaw portion 14, the cutting cutter 15 and the lower jaw portion 116 in the above-mentioned forceps 1, 1X, 1Y.

FIG. 23 at (a) shows a front view of the forceps 1 in which the upper jaw portion 14, the cutter 15, and the lower jaw portion 116 are not provided with the coaxial electrode 20 or the irradiation electrode 24. FIG. 23 at (b) shows a front view of the forceps 1X in which the upper jaw portion 14, the cutter 15, and the lower jaw portion 116 are not provided with the coaxial electrode 20 and the irradiation electrode 24, and FIG. 23 at (c) shows a front view of the forceps 1Y in which the coaxial electrode 20 and the irradiation electrode 24 are not provided on the upper jaw portion 14, the cutter 15, and the lower jaw portion 116.

FIG. 24 is a schematic diagram of a confirmation test in which solidification due to microwaves irradiated from the coaxial electrode 20 is confirmed. Specifically, FIG. 24 at (a) to (e) shows the test situation in which microwaves were irradiated from the coaxial electrodes 20 provided on the fixed blade 119b and the movable blade 14b of the end effector 10b in the egg white and the coagulated egg white M is cut. FIG. 24 at (f) to (h) shows the test conditions in the case where the coaxial electrode 20 is provided only on the lower jaw portion 116a of the end effector 10a, that is, the coaxial electrode 20 is provided on the so-called single-edged blade.

FIG. 24 is a schematic diagram of a confirmation test in which solidification due to microwaves irradiated from the coaxial electrode 20 is confirmed. Specifically, FIG. 24 (a) to (e) shows a test condition in the case where the coaxial electrode 20 is provided only on the lower jaw portion 116a of the end effector 10a, that is, the coaxial electrode 20 is provided on the so-called single-edged blade. FIG. 24 (f) to (h) shows at test condition in the case where the coaxial electrode 20 is provided only on the lower jaw portion 116a of the end effector 10a, that is, the coaxial electrode 20 is provided on the so-called single-edged blade.

As shown in FIG. 24 at (a), an end effector 10b having a coaxial electrode 20 on both blades is disposed in egg white, a microwave irradiation from the coaxial electrode 20 is provided on both blades by activating microwave generator 30. Then, as shown in FIG. 24 at (b), coagulation starts from the base side where the distance between the coaxial electrodes 20 is narrow between the fixed blade 119b and the movable blade 14b constituting the cutting mechanism 19, and the coagulated egg white M is formed.

When microwave irradiation from the coaxial electrodes 20 provided on both blades is continued, as shown in FIG. 24 at (c), the coagulated egg white M expands along the coaxial electrodes 20 provided on the fixed blade 119b and the movable blade 14b.Then, when the movable blade 14b is rotated with respect to the fixed blade 119b constituting the cutting mechanism 19, the coagulated egg white M may be cut by the cutting blade provided on the fixed blade 119b and the movable blade 14b as shown in FIG. 24 at (d).

When cutting the coagulated egg white M with the cutting blade, the coagulated egg white M may be cut while irradiating the microwave from the coaxial electrode 20 provided on the fixed blade 119b and the movable blade 14b, or the coaxial electrode 20 does not irradiate the microwave.

Then, as shown in FIG. 24 at (e), the movable blade 14b constituting the cutting mechanism 19 is opened, the coagulated egg white M is advanced in the direction of the arrow with respect to the cut portion, and the microwave is irradiated again from the coaxial electrode 20. As shown in FIG. 24 at (b), coagulated egg white M is formed. By repeating this, it was confirmed that the cutting mechanism 19 in the end effector 10b may cut, for example, the coagulated portion in the blood vessel B. It is presumed that this result will be an operation to strengthen the coagulation function and suppress the deterioration of the hemostatic function in the blood clot due to bleeding.

When the coaxial electrode 20 is provided on both the lower jaw portion 116a and the upper jaw portion 14a of the end effector 10a, or when the coaxial electrode 20 is provided on the lower jaw portion 116a and the cutting cutter 15a, or further when the coaxial electrode 20 is provided in all of the lower jaw portion 116a, the upper jaw portion 14a, and the cutting cutter 15a, similar to the end effector 10b provided with the coaxial electrode 20 on both blades described above, the cutting mechanism 19 in the end effector 10a may cut, for example, the coagulated portion of the blood vessel B.

On the other hand, in the case of the end effector 10a, which is a single-edged electrode in which the coaxial electrode 20 is provided only on one of the lower jaw portion 116a and the upper jaw portion 14a constituting the cutting mechanism 19, the end effector 10a is as shown in FIG. 24 at (g). The coagulated egg white M is formed by irradiating the coaxial electrode 20 provided on the single-edged blade with microwaves, and the coagulated egg white M enlarged along the coaxial electrode 20 provided on the single-edged blade may be cut by the cutter 15a.However, as shown in FIG. 24 at (c), the formation rate of the coagulated egg white M is slower and the size of the coagulated egg white M formed is smaller than that in the case where the coaxial electrode 20 is provided on both blades.

Surgical equipment such as end effectors 10a and end effectors 10b that irradiate microwaves emit less smoke and mist, have a strong hemostatic function, and are useful for surgical support in closed spaces such as endoscopic surgery and robotic surgery. However, when there is a lot of bleeding, hemostasis in the blood pool and hemostasis in the liquid are diminished like other energy devices, so that it is resolved by irradiating both blades with microwaves..

In the above-mentioned effect confirmation test, although not shown, the center conductor 21 of the coaxial electrode 20 and one of the semicircular conductors 23 are arranged on one of the double-edged blades to form an electrode, and the other of the double-edged blades is the center of the coaxial electrode 20. In the case of an end effector in which the other conductor of the conductor 21 and the semicircular conductor 23 is arranged and used as an electrode, the microwave is emitted from one electrode of the double-edged blade toward the electrode provided on the other, so that the end effector opens in the air. Although coagulation starts from the middle part of the tissue sandwiched between the blades, coagulation proceeds only to the electrode part on the side irradiated with microwaves in the egg white, and the rate of coagulation of the surrounding egg white at the other electrode is considerably delayed. It has been also confirmed that the blood-stopping power is weakened.

On the other hand, as described above, when the coaxial electrodes 20 provided on each of the double-edged blades of the end effector 10b irradiate microwaves, solidification proceeds from the coaxial electrodes 20 provided on the double-edged blades (see FIG. 24 at (b) and (c)), The egg white coagulated and the coagulated egg white M could be formed after twice the time in the air. Further, in the case of the end effector 10a having the coaxial electrode 20 on only one of the two blades, the coagulated egg white M was formed, but the formation of the coagulated egg white M on the entire surface was not completed even after taking three times as long. As described above, the arrangement of the coaxial electrode 20 that affects the formation of the coagulated egg white M may be selected according to the usage environment and the treatment target.

FIG. 25 shows a medical device 220 according to another embodiment of the present invention. In FIG. 25 at (a), the medical device 220 includes a surgical device 221 that drives the end effector 10a of the multifunctional surgical device (medical processing tool) as described with FIGS. 16 to 24. Instead of the end effector 10a, the medical device 220 may be provided with the end effector 10b.

The surgical apparatus 221 includes a microwave irradiation module 222 including a microwave control circuit such as an irradiator and an amplifier, and a driving unit 224 of a mechanical mechanism that drives the end effector 10c by manual operation of the lever 223. Specifically, the trigger handle 13 of the forceps shown in FIGS. 1 to 15 may be used as the lever 223, and the mechanical mechanism from the trigger handle 13 to the end effector 10a, 10b may be incorporated into the surgical apparatus 221.

The microwave irradiation module 222 having an irradiator is provided in the surgical apparatus 221 but, if necessary, inside the shaft portion 225 or in the bent portion 226 having a wrist function (such as the flexible portion 101 in FIG. 16). Alternatively, by providing the end effector 10a (FIGS. 1 and 2, or the electronic module 31 of FIG. 16), the medical device 220 may be miniaturized.

The driving unit 224 operates the end effector 10a of FIG. 16 via the shaft portion 225 by the operator manually gripping and operating the lever 223 of the gripping member of the surgical apparatus 221.

The surgical apparatus 221 receives power from the adapter 227.The microwave irradiation module 222 included in the surgical apparatus 221 may be configured to be directly or indirectly connected to an irradiator installed in the robot body in addition to the conventional stationary type, shoulder-mounted type, and built-in type.

In the above embodiment, the microwave irradiation does not coagulate when gripped by the lower jaw portion 116 and the upper jaw portion 14 of the end effector 10a, but at the target portion when the upper jaw portion 14 rotates with respect to the lower jaw portion 116. Even if a certain biological tissue is coagulated, the coagulation and hemostasis of the tissue can be cut. Further, when the biological tissue is gripped by the gripping mechanism 17a in the end effector 10a and cut by the cutter 15, microwaves may be irradiated.

With such forceps, the tissue may be opened and removed, griped to remove obstacles, and the gripping function can be used for the operation of maintaining the surgical field. Moreover, the part to be coagulated and cut can be griped with forceps and coagulated and cut as it is. The operations of coagulation and cutting do not require re-gripping or re-pinching a plurality of tissues, but the operation is completed by gripping the pinched tissue once. Therefore, surgical operations can be performed with one device for gripping, coagulation, and cutting.

It is all the operation of general extraction surgery, and the extraction operation may be completed with a single instrument, and there is no need to replace it with another instrument.

As a modification of this embodiment, the microwave irradiation module 222 may be provided with an execution program memory, and the driving unit 224 may be an electric mechanical mechanism controlled by the execution program. As an operation, when the lever 223 is gripped, the execution program actuates the driving unit 224 based on the position data of the lever 223, and by forming the end effector 10a in FIG. 1 and moving the slide frame 12, the upper jaw portion 14 is rotated in the direction toward the lower jaw portion 116 in cooperation with the cutting cutter 15 (see FIG. 2), a microwave signal is sent to the irradiation electrode 24 by the above program, and the living tissue is irradiated with microwaves to be coagulated.

When the lever 223 is further gripped, the movable frame 12a is further advanced by the program according to the position data as described above, and the living tissue is gripped by the upper jaw portion 14a and the lower jaw portion 116a.

When the lever 223 is further gripped, the movable frame 12a further advances according to the above program according to the position data of the lever and rotates only the cutting cutter 15 to cut the coagulated living tissue. By releasing the lever 223 after cutting, the medical device 220 is configured to return to the initial state (open state) based on the position data of the lever.

FIG. 25 at (b) is a schematic diagram of a surgical system provided with a plurality of medical devices 220 illustrated in FIG. 25 at (a), and the internal configuration of each is the same as that of FIG. 25 at (a), but the microwave irradiation module 222 of the medical device 220 further includes a unit that synchronizes the wavelengths of the microwaves of each other, and synchronizes via the adapter 227 or wirelessly.

Therefore, when two medical devices 220 are operated in a patient by one or two operators, the wavelengths of both microwaves are synchronized, so that sparks or the like occurring between the two medical devices 220 may be prevented and safety is increased.

FIG. 26 shows the remote surgery system 200 in one embodiment of the present invention. The remote surgery system 200 is provided with a surgeon console 201 that serves as a station for each of the two operators D (D1 and D2), a master control unit 202 operated by the operator D, a viewing angle-core cart 240, and a patient-side cart having a robot of patient-side cart 210.

The surgeon console 201 includes a viewer 201a in which an image of the surgical portion is displayed on the operator D. When using the surgeon console 201, operators D1 and/or D2 generally sit in the chair of the surgeon console, align their eyes in front of the viewer 201a, and grab the master control unit 202 in one hand.

In the remote surgery system 200, two operators can operate at the same time, but even one operator can operate it. When two operators operate at the same time, the two operators can operate in cooperation with each other, which has an advantage that the operation time of the entire patient can be shortened. The surgeon console 201 and the master control unit 202 may be provided in a system in which three or more units are provided, if necessary.

The robot of the patient-side cart 210 is arranged adjacent to the patient. During use, the patient-side cart 210 is placed near the patient in need of surgery. The robot of the patient-side cart 210 is provided with casters on the pedestal 211 so that it can be fixed but moved during surgery. The surgeon console 201 is used in the same operating room as the patient cart, but may be installed remotely from the patient cart 210.

The patient-side cart 210 includes four robot arm assemblies 212, but the number of robot arm assemblies 212 is arbitrary. Each robot arm assembly 212 has a structure that is connected to a drive device 213 that enables three-dimensional movement and is driven and controlled.

A display 214 displays image data related to surgery. The drive device 213 is controlled by the master control unit 202 of the surgeon console 201. The movement of the manipulator portion of the robot arm assembly 212 is controlled by the operation of the master control unit 202.

An image acquisition device 215 such as an endoscope is arranged in the robot arm assembly 212a, which is one of the four robot arm assemblies 212. A visual camera 216 is included at the remote end of the image capture device 215. The elongated shaft-shaped image capture device 215 allows the visual camera 216 to be inserted through the surgical entry port of the patient (not shown).

The image capture device 215 is operably connected to the viewer 201a of the surgeon console 201 to display the image captured by its visual camera 216.

Each of the other robot arm assemblies 212 is a linking device that supports and includes tools 217, which are removable surgical instruments, respectively. The tools 217 of the robot arm assembly 212 each include an end effector 10c.

Tool 217 has an elongated shaft that allows the end effector 10a to be inserted through the patient's surgical entry port. The movement of the end effector 10c is controlled by the master control unit 202 of the surgeon console 201.

When a microwave irradiation electrode (20, 24) and a microwave irradiation unit are used for the end effector 10a (10b) as the tool 217, the wavelengths of the microwaves emitted from the end effector 10c are synchronized with each other.

For example, when one or two operators D operate a plurality of tools 217 in a patient, the wavelengths of the irradiated microwaves are synchronized, so that the plurality of tools 217 or a plurality of end effectors 10a (10b) are synchronized. It is possible to prevent the occurrence of sparks between them, and safety is enhanced. Operating a plurality of end effectors 10a (10b) at the same time can shorten not only advanced surgery but also surgery time.

FIG. 27 shows the configuration of a tool 217 that is mounted on the robot arm assembly 212 of the remote surgery system 200 of FIG. 26 as a representative example of a surgical device. The tool 217 mounted on the other robot arm assembly 212 may have the same configuration or may be a surgical device having another configuration.

FIG. 27 at (a) shows a plane view of the tool 217. The tool 217 has an end effector 10a of a multifunctional surgical device, a bending portion 226, a shaft portion 225, a surgical device 221 for driving, controlling and monitoring the tool 217, and a connector 228 for connecting to a robot. The bent portion 226 increases the degree of freedom in the operation angle of the effector, and improves the accuracy of robot control.

FIG. 27 at (b) shows the internal configuration of the tool 217 of FIG. 47 at (a). FIG. 16 shows a medical system composed of a surgical device 221 that drives an end effector 10c directly connected to a slide shaft via a shaft portion 225 and a robot of a patient-side cart 210 that controls the surgical device 221.

The end effector 10a includes a cutting device having a rotatable upper jaw portion 14 and a lower jaw portion 116 held openable and closable, and a movable cutter 15 attached to the upper jaw portion 14 and the lower jaw portion 116. The surgical apparatus 221 connected to the end effector 10a is a matching unit with the tool 217, a reflected wave monitor 232, a control circuit for controlling signals in the surgical apparatus 233, and a cutting device of the tool 217 via a shaft portion 225 of the tool 217. It has an irradiation / driving unit 234 having an amplifier for mechanically driving the operation and an oscillator generating microwaves, and a signal interface 235 with the robot of the patient-side cart 210.

As described in FIG. 28, the robot of the patient-side cart 210 is connected to the surgical device 221 via the signal interface 235 and the connector 228 by wire and/or wirelessly, and is connected to the robot of the patient-side cart 210. Inside, an input unit 210a that receives an operation signal from the master control unit 202, an arithmetic unit CPU that executes a predetermined operation program based on the operation signal, and a surgical apparatus 221 based on the output from the arithmetic unit. An output unit 210a that generates a drive signal for driving the upper jaw portion 14 and the cutter 15 of the end effector 10c is provided. The input unit and the output unit are composed of an input/output unit 210a (I/O).

FIG. 28 is an explanatory diagram of the remote surgery system 200, FIG. 28 at (a) is a block diagram showing a connection relationship with each unit, and FIG. 28 at (b) is an operation flow diagram of the remote surgery system 200.

The visual-core cart 240 has functions related to the image acquisition device. When the remote surgery system 200 is activated for surgery, the surgeon operates the master control unit 202 of the surgeon console 201, and if there are two surgeons, also operates the master control unit 202 of the surgeon console 201 (step S1). The command generated by the operation is transmitted to the visual core cart 240 (step S2).

The visual core cart 240 then interprets the signal and moves the desired robot arm assembly 212 to the patient's surgical area (step S3).

Next, the tool 217 attached to the selected robot arm assembly 212 is inserted into the patient through an elongated pipe (step S4), and the end effector 10c of the above embodiment is operated to grip, coagulate, and cut the tissue. (Step S5), the operation of the living tissue is completed.

The operation of gripping, coagulating, and cutting the biological tissue in step S5 is the operation of the end effector 10a (10b), but there are three operation patterns: a first operation of coagulating, gripping, and cutting the biological tissue while irradiating microwaves from the electrode; a second operation of gripping a living tissue, irradiating microwaves from the electrode to the living tissue to coagulate after gripping, and cutting while irradiating the microwaves; and a third operation in which the irradiation of microwaves from the electrode is stopped and cut the living tissue though the living tissue is gripped and coagulated while irradiating the microwaves to the living tissue from the electrode. These three movement patterns are configured to be selectively available according to the surgical content. Further, it may be a pattern that is shared and operated by a plurality of tools of FIG. 26, such as being gripped or solidified by the tool 217 and cut by another tool 217.

The operation of coagulation/gripping/crashing/cutting of the tissue in this embodiment may obtain same control operation flow by operating the end effector 10a (10b) based on the signal from the robot 210 instead of the control operation of the end effector 10a (10b) based on the position data of the lever 223 described as a modification of the embodiment of FIG. 25.

The above-mentioned examples and embodiments have been described for medical treatment such as forceps and medical instruments, but the present invention is not limited to the above description, and the target site is not only a part of an object but also a member. That is, the term "target site" shall include any "target portion". In other words "target portion" shall include any "target site". It may include a general-purpose cutting device capable of gripping/contacting and cutting the target member. Further, the energy wave irradiated from the electrode of the forceps is not limited to the microwave, and may include other electromagnetic waves and electric currents.

For example, as described above, in the cutting mechanism 10 and the end effector 10a of the forceps 1 and the like, the gripping mechanism 17 is configured by the lower jaw portion 116 and the upper jaw portion 14, and the target portion such as the blood vessel B is gripped by the gripping mechanism 17 which is configured to cut the target portion by the cutter 15, but as shown in FIG. 6 at (d), the gripping mechanism 17 may be composed of the lower jaw portion 116 and the upper jaw portion 14 provided on both sides of the cutter 15 and is provided. The target portion during gripping by the set gripping mechanism 17 may be designed to be cut by the cutter 15.

Further, the horizontal movement of the forceps or the end effector of the present invention is not limited to the axial movement, and may be a structure connected by a wire in order to increase the degree of freedom in the position and angle of the end effector. The spring mechanism for moving the upper jaw portion and the cutter for gripping, coagulating, and cutting the living tissue is not limited to the embodiment, and may be provided at any position.

Further, other than the electrodes and the cutting edge, insulation coating may be appropriately performed to prevent corrosion and sparks. In the above embodiments, the tissue between the upper jaw portion 14 and the cutter 15 and the lower jaw portion 116 can be coagulated from both sides. The split portion of the coaxial cable that supplies microwaves to the end effector enables a structure that does not restrict the movement of multiple joints and sends energy beyond multiple robot joints with a flexible cable.

Also, by using a metal blade, the rigidity and shape can be freely designed. The medical device having the forceps and end effector of the present invention can be used under MR image guidance, and microwave energy can be introduced not only under a microscope or a robot hand but also into endovascular surgery and fetal surgery.

The horizontal movement of the forceps or end effector of the present invention is not limited to axial movement, and may be connected by a wire in order to increase the degree of freedom in the position and angle of the end effector.

The spring mechanism for moving the upper jaw portion and the cutter for gripping, coagulating, and cutting the living tissue is not limited to the above embodiments and may be provided at any position. Further, other than the electrodes and the cutting edge, insulation coating may be appropriately performed to prevent corrosion and sparks.

In the above embodiments, the tissue between the blades of both gripping members (upper and lower jaws, double blades) may be coagulated from both sides. The split portion of the coaxial cable that supplies microwaves to the end effector makes it possible to send energy beyond multiple robot joints with a flexible cable without restricting the movement of multiple joints.

In the above embodiment, the tissue between the upper jaw portion 14 and the cutter 15 and the lower jaw portion 116 may be coagulated from both sides. The split portion of the coaxial cable that supplies microwaves to the end effector makes it possible to send energy beyond multiple robot joints with a flexible cable without restricting the movement of multiple joints.

Also, by using a metal blade, the rigidity and shape can be freely designed. The medical device having the forceps and end effector of the present invention can be used under MR image guidance, and microwave energy can be introduced not only under a microscope or a robot hand but also into endovascular surgery and fetal surgery.

The functional word of gripping is used in the president embodiments, but the gripping may include the operation of grasping after gripping.

The embodiments of the present invention are exemplified in all respects, and the scope of the present invention includes all modifications within the meaning and scope equivalent to the scope of claims.

### [Explanation of symbols]

1, 1X, 1Y ... Forceps
10, 10X ... Cutting mechanism
10a, 10b ... End effector
11, 11X, 11Y ... Body frame
11a, 11b ... Reference axis
12 ... Upper slide axis
12a, 12b ... Movable shaft
12Xa, 12Ya ... First slide plate
12Xb, 12Yb ... Second slide plate
13, 13X, 13Y ... Trigger handle
14, 14a ... Upper jaw portion
14b ... Movable blade
15, 15a ... Cutter
15Y ... Slide cutter
16 ... Spring
16X, 16Y ... Coil spring
17, 17a ... Gripping mechanism
18Y ... Arm
19 ... Cutting mechanism
20 ... Coaxial electrode
21... Central conductor
22 ... Semi-circular insulator
23 ... Semicircular tube conductor
24a, 24b, 24c ... (irradiation) electrodes
30 ... Microwave generator
31 ... Electronic module
35 ... Core cart
40 ... Coaxial cable
41 ... Central conductor
42 ... Insulator
43 ... Outer conductor
44 ... Insulation coating
100 ... Support
101 ... Flexible part
111 ... Fixed handle frame
112, 112X, 112Y ... Reference frame
112Xa ... Slide frame
113, 131 ... Ring part
114 ... Corner
115 ... Support shaft
116, 116a ... Lower jaw portion
117, 117b ... Distal end support shaft
118, 123 ... Click convex part
119b ... Fixed blade
121, 121Ya, 121Yb ... Upper support shaft
121X ... Middle support shaft
122, 122Xa, 122Yb ... Support shaft on the tip
122Xb ... Contact ring part
123X ... Regulation hole
124Y ... Regulation frame
132 ... Pivot portion
133 ... Upper pivot portion
134 ... Upper stage pivot portion
135X ... Pressing block
141, 141b, 151 ... Shafted portion
142, 152 ... Rotating shaft
143 ... Projection control section
153, 153a, 153b, 153Y ... Cutting blade
161X ... Insertion shaft
162X ... Flange part
181Y ... Drive shaft
200 ... Remote surgery system
201 ... Surgeon console
201a ... Viewer
202 ... Master control unit
210 ... Patient cart
210a ... Input / output unit
211 ... pedestal
212, 212a ... Robot arm assembly
213 ... Drive device
214 ... Display
215 ... Image capture device
216 ... Visual camera
217 ... Tool
220 ... Medical equipment
221 ... Surgical equipment
222 ... Microwave irradiation module
223 ... lever
224 ... Drive unit
225 ... Shaft part
226 ... Bent part
227 ... Adapter
228 ... Connector
231 ... Matching unit
232 ... Reflected wave monitor
233 ... Control circuit
234 ... Irradiation / drive unit
235 ... Signal interface
240 ... Core cart
B ... Blood vessels
D, D1 ... Operator
F ... Distal end side
L ... Longitudinal direction
M ... Coagulated egg white
R ... Base end side

## Claims

1. A cutting device comprising:
a first contact member and at second contact member assembled so as to be openable and closable,
a contact mechanism that rotates the first contact member toward the second contact member to bring the first contact member and the second contact member into contact with a target portion, and
a cutting mechanism for cutting the target portion in a state where the first contact member and the second contact member are in contact with the target portion by the contact mechanism.

2. The cutting device according to claim 1, further comprising:
a gripping mechanism in which the first contact member and the second contact member are a first gripping member and a second gripping member that grip the target portion and the contact mechanism is constructed to grip the target portion, and a cutting member attached to the first gripping member,
wherein the cutting mechanism moves the cutting member along the first gripping member in a gripping state where the first and second gripping members grip, and cuts the target t by coming in contact with the second gripping member.

3. The cutting device according to claim 2, wherein the cutting mechanism in a state where the target portion is gripped by the gripping mechanism is constructed to rotate the cutting member in the same direction as the rotation of the first gripping member, and cut the target portion by contacting the second gripping member.

4. The cutting device according to claim 3, wherein the first gripping member is provided with a protrusion restricting portion that regulates the cutting member that rotates in the same direction to protrude beyond the first gripping member in the direction opposite to the cutting direction.

5. The cutting device according to claim 3, wherein the cutting mechanism in a state where the target portion is gripped by the gripping mechanism is constructed to slide the cutting member along the second gripping member, and cut the target portion by the cutting member.

6. The cutting device according to any one of claims 2 to 5, further comprising electrodes for irradiating electromagnetic waves to at least one of a gripping portion where the gripping member grips the target portion and a vicinity of a cutting blade in the cutting member, and a gripping part where the second gripping member that grips the target portion.

7. The cutting device according to claim 6,
wherein at least one of the electrodes is a coaxial electrode provided with a center electrode and an outer electrode surrounding the center electrode via an insulator, and
wherein a plurality of coaxial cables connecting an irradiation device for irradiating the electromagnetic waves and the electrodes are branched in parallel, and each of the coaxial electrodes is electrically connected to the central conductor and the outer conductor of the coaxial cable.

8. The cutting device according to claim 7, wherein the coaxial electrodes are connected in the opposite polarity to the coaxial cable.

9. A forceps having the cutting device according to any one of claims 2 to 5 in which the target portion is a living tissue, and the first gripping member and the second gripping member are a first jaw member and a second jaw member, respectively, further comprising:
a driving mechanism that grips and coagulates the biological tissue by rotating the first jaw member toward the second jaw member, and
one operation member operating the driving mechanism and the cutting mechanism,
wherein at least a part of the living tissue is coagulated by irradiating electromagnetic waves from the electrodes, and
wherein the biological tissue is gripped by the first jaw member and the second jaw member, and then cut by the cutting mechanism in accordance with a series of operations of the one operation member.

10. A forceps having the cutting device according to any one of claims 6 to 8 in which the target portion is a living tissue, and the first gripping member and the second gripping member are a first jaw member and a second jaw member, respectively, further comprising:
a driving mechanism that grips and coagulates the biological tissue by rotating the first jaw member toward the second jaw member, and
one operation member operating the driving mechanism and the cutting mechanism,
wherein at least a part of the living tissue is coagulated by irradiating electromagnetic waves from the electrodes, and
wherein the biological tissue is gripped by the first jaw member and the second jaw member, and then cut by the cutting mechanism in accordance with a series of operations of the one operation member.

11. The forceps according to claim 9 or 10, further including a differential mechanism which operates the gripping mechanism and the cutting mechanism together by the operation of the operation until the target portion is gripped, and differentially operates the cutting mechanism relative to the gripping mechanism in a gripping state in which the target portion is gripped by further operation of the operation member.

12. The forceps according to claim 11 further including a notification mechanism disposed to notify a user of the operation of the differential mechanism by a series of operations of the operation member.

13. The cutting device according to claim 1 in which the first contact member and the second contact member are a first cutting member and a second cutting member provided with a cutting blade for cutting the target portion at a contact portion that contacts the target portion, and the contact mechanism is the cutting mechanism that cuts the target portion by contact with the first cutting member and the second cutting member, further comprising:
electrodes for irradiating electromagnetic waves along the cutting blades of the first cutting member and the second cutting member,

14. The cutting device according to claim 13, wherein the electrodes including a coaxial electrode provided with a center electrode and an outer electrode surrounding the center electrode via an insulator, and wherein a coaxial cable connecting the irradiation device irradiating the electromagnetic wave and the electrodes is branched to a plurality of coaxial cables in parallel such that each of the coaxial electrodes is electrically connected to the central conductor and the outer conductor of the coaxial cable.

15. The cutting device according to claim 14, wherein the coaxial electrodes are connected in the opposite polarity to the coaxial cable.

16. A forceps provided with the cutting device according to any one of claims 13 to 15, wherein the target portion is a living tissue, and the first contact member and the second contact member are a first jaw member and a second jaw member, respectively, further including;
a driving mechanism that contacts the living tissue by rotating the first jaw member toward the second jaw member to coagulate and cut the living tissue, and
an operation unit for operating the drive mechanism,
wherein by a series of operations of the operation unit, the first jaw member and the second jaw member are brought into contact with the living tissue to coagulate the living tissue, and to cut the same by the cutting mechanism.

17. A surgical system provided with a plurality of medical devices having the forceps according to claim 10 or 16,
wherein the electrode provided on each of the forceps is an electrode for microwave irradiation, and each of the forceps is provided with a microwave irradiation unit, and
wherein a period of the microwave applied from the coaxial cable to each electrode is the same.

18. A medical system comprising:
the forceps according to any one of claims 9, 10, and 16 a driving unit that drives the gripping mechanism and the cutting mechanism of the forceps, and
a medical robot connected to the driving unit to apply a drive signal to the same.

19. A grip and cut method using the cutting device according to any one of claims 2 to 5, comprising the steps of
rotating the first gripping member toward the second gripping member and gripping the target portion between the first gripping member and the second gripping member, and
moving the cutting member attached to the first gripping member along the first gripping member and contacting with the second gripping member to cut the gripped target portion.

20. The grip and cut method of claim 19, wherein in the cutting step, the cutting member attached to the first gripping member is rotated in the direction of the second gripping member, or is slid forward along the second gripping member.

21. An end effector comprising:
a movable upper jaw member rotatably connected to a shaft,
a stationary lower jaw member supported by a main body frame,
a rotatable cutting member attached to the upper jaw member and engaged with the upper jaw member and the lower jaw member,
a first electrode provided on the surface of the upper jaw member facing the lower jaw member,
a second electrode provided on the surface of the lower jaw member facing the upper jaw member, and
a third electrode provided at or near the cutting edge of the cutting member,
a coaxial cable connected to the first electrode, the second electrode, and the third electrode, and
a flexible member with a bendable cover shape.

22. A robot comprising:
an input/output unit wired and/or wirelessly connected to the cutting device according to any one of claims 1 to 8,
an input unit to receive an operation signal in real time,
an arithmetic unit to execute a predetermined operation program based on the operation signal, and
an output unit to generate a drive signal based on an output from the arithmetic unit to contact and/or cut the target portion by the first contact member and the second contact member.

23. A robot comprising:
an input/output unit wired and/or wirelessly connected to the cutting device according to any one of claims 6 to 8, and 13 to 15,
an input unit to receive an operation signal in real time,
an arithmetic unit to execute a predetermined operation program based on the operation signal, and
an output unit to generate a drive signal based on an output from the arithmetic unit to contact and/or cut the target portion by the first contact member and the second contact member.

24. A surgical medical robot provided with the robot according to claim 22 or 23, wherein the output unit provides a drive signal to an externally provided drive unit that mechanically drives the device.

25. A robot control method comprising the steps of
generating commands by operating the master control unit,
moving the robot arm assembly to the treatment position by the above command,
moving the tool attached to the robot arm assembly to the treatment position, and
controlling the movement of the forceps according to one of claims 9, 10, and 16 attached to a distal end of the tool, and its electromagnetic wave irradiation.

26. A surgical system comprising:
a plurality of surgeon consoles that serve as stations for a plurality of operators;
a master control unit operated by the plurality of operators; and
the robot according to claim 22 or 23, which is a patient-side cart.

27. A cutting device comprising;
one operation unit,
a first gripping member and a second gripping member assembled so as to be openable and closable,
a cutting member attached to the first gripping member,
a gripping mechanism that rotates the first gripping member toward the second gripping member and grips the living tissue by the first gripping member and the second gripping member in response to the movement of the one operation unit in a state where the living tissue is gripped by the gripping mechanism, and
a cutting mechanism for moving the cutting member along the first gripping member in response to further movement of the operating portion and cutting the gripped living tissue by contacting the second gripping member.

28. The cutting device according to claim 27comrising a differential mechanism in which the gripping mechanism and the cutting mechanism both operate until the target portion is gripped in response to an operation of the one operation unit, and the cutting mechanism differentially operates from the gripping mechanism in a gripping state in which the target portion is gripped by further operation of the operation unit.
